# EUROPEAN PATENT SPECIFICATION

(11) **EP 1 506 782 B1**
(45) Date of publication and mention of the grant of the patent: **03.10.2007**
(21) Application number: 03254976.8
(22) Date of filing: 11.08.2003
(51) Int. Cl.: A61K 36/48, A61P 17/00

(54) **Vapor fraction from seeds of glycine max (L.) merr. and composition thereof**
Dampf-Fraktion von Glycine max (L.) merr. Samen und entsprechende Zusammensetzung
Fraction de vapeur de graines de glycine max (L.) merr. et composition correspondante

(43) Date of publication of application: 16.02.2005
(73) Proprietor: Chu, I-Hung, Kaohsiung County (TW)
(72) Inventor: Chu, I-Hung, Kaohsiung County (TW)
(74) Representative: Sexton, Jane Helen

(56) References cited:
- US-B1- 6 248 910
- PATENT ABSTRACTS OF JAPAN vol. 008, no. 106 (C-223), 18 May 1984 (1984-05-18) & JP 59 021364 A (AJINOMOTO KK), 3 February 1984 (1984-02-03)
- DATABASE WPI Section Ch, Week 199436 Derwent Publications Ltd., London, GB; Class B04, AN 1994-290827 XP002283271 & JP 06 219926 A (TOYAMA CHEM CO LTD) 9 August 1994 (1994-08-09)
- DATABASE WPI Section Ch, Week 200351 Derwent Publications Ltd., London, GB; Class D13, AN 2003-536380 XP002269467 & JP 2003 088320 A (HOYA NATTO KK) 25 March 2003 (2003-03-25)
- PATENT ABSTRACTS OF JAPAN vol. 2000, no. 09, 13 October 2000 (2000-10-13) & JP 2000 169347 A (BUN JUNSAI), 20 June 2000 (2000-06-20)
- DATABASE WPI Section Ch, Week 200114 Derwent Publications Ltd., London, GB; Class D13, AN 2001-128228 XP002269468 & JP 2000 316473 A (NISSHIN OIL MILLS LTD) 21 November 2000 (2000-11-21)
- PATENT ABSTRACTS OF JAPAN vol. 1997, no. 10, 31 October 1997 (1997-10-31) & JP 09 143087 A (SANSHO SEIYAKU CO LTD), 3 June 1997 (1997-06-03)
- PATENT ABSTRACTS OF JAPAN vol. 1999, no. 13, 30 November 1999 (1999-11-30) & JP 11 206342 A (RYUEI SOKEN:KK), 3 August 1999 (1999-08-03)
- DATABASE WPI Section Ch, Week 200271 Derwent Publications Ltd., London, GB; Class B04, AN 2002-658445 XP002269469 & CN 1 188 001 A (AN F) 22 July 1998 (1998-07-22)
- DATABASE WPI Section Ch, Week 200029 Derwent Publications Ltd., London, GB; Class B05, AN 2000-331699 XP002269470 & JP 2000 080029 A (SUNSTAR CHEM IND CO LTD) 21 March 2000 (2000-03-21)
- DATABASE EPODOC EUROPEAN PATENT OFFICE, THE HAGUE, NL; 19 February 2003 (2003-02-19), WANG GUANGSHUN: "Prescription and preparing process for compound spanishneedles ointment" XP002271606 & CN 1 397 321 A (WANG GUANGSHUN) 19 February 2003 (2003-02-19)
- DATABASE WPI Section Ch, Week 199834 Derwent Publications Ltd., London, GB; Class B04, AN 1998-393396 XP002269471 & JP 10 158177 A (KAO CORP) 16 June 1998 (1998-06-16)
- DATABASE WPI Section Ch, Week 198501 Derwent Publications Ltd., London, GB; Class A96, AN 1985-003536 XP002269472 & JP 59 204120 A (NITTO ELECTRIC IND CO) 19 November 1984 (1984-11-19)
- PATENT ABSTRACTS OF JAPAN vol. 013, no. 562 (C-665), 13 December 1989 (1989-12-13) & JP 01 233207 A (CHIYUUWA INTERNATL:KK), 19 September 1989 (1989-09-19)
- PATENT ABSTRACTS OF JAPAN vol. 1996, no. 05, 31 May 1996 (1996-05-31) & JP 08 026937 A (DAIMU INTERNATL KK), 30 January 1996 (1996-01-30)
- DATABASE WPI Section Ch, Week 200278 Derwent Publications Ltd., London, GB; Class D21, AN 2002-720156 XP002283272 & KR 2002 027 760 A (KOREAN COSMETICS CO LTD) 15 April 2002 (2002-04-15)
- PATENT ABSTRACTS OF JAPAN vol. 2002, no. 10, 10 October 2002 (2002-10-10) & JP 2002 179529 A (POLA CHEM IND INC), 26 June 2002 (2002-06-26)
- DATABASE WPI Section Ch, Week 200348 Derwent Publications Ltd., London, GB; Class B04, AN 2003-511186 XP002283273 & KR 2003 021 811 A (KIM J Y) 15 March 2003 (2003-03-15)
- DATABASE EPODOC EUROPEAN PATENT OFFICE, THE HAGUE, NL; 7 July 1993 (1993-07-07), YANG YANJUN: "Local dressing for treatment of appendicitis and preparation method" XP002283428 & CN 1 073 876 A (YANG YANJUN) 7 July 1993 (1993-07-07)

## Description

### BACKGROUND OF THE INVENTION

### 1. Field of the invention

The invention mainly relates to a vapor fraction from the seeds of *Glycine max* (L.) Merr. which has effects in treating skin injuries, in treating dermatological disorders, in stimulating cell regeneration, and in stimulating hair growth.

### 2. Description of the Related Art

*Glycine max* (L.) Merr., including soybean and black soybean, is one of the most important sources of oil and proteins in the world. For instance, soybeans can be processed to obtain an edible, semi-drying oil that is used as salad oil, or for manufacture of margarine and shortening. Soybean oil can be also used in the manufacture of paints, linoleum, oilcloth, printing inks, soaps, insecticides, and disinfectants. Besides, lecithin phospholipids obtained from the by-products of the oil industry, can be used as wetting and stabilizing agents in food, cosmetics, pharmaceuticals, leathers, paints, plastics, soaps, and detergents. Soy meal is a very protein-rich feeding stuff for livestock. In addition, soybean protein can be used in manufacture of synthetic fibers, adhesives, textile sizing, waterproofing, fire-fighting foams and so on.

In medical use, soybeans have been reported to have effects on many diseases.

Soybean can be used as a nutritious supplement for regulating the functions of bowels, heart, kidney, liver, and stomach. Since soybean oil contains a high amount of unsaturated fatty acids, it can be used to combat hypercholesteremia. Medical lecithin from soybeans functions as a lipotropic agent. In addition, tigmasterol known as an antistiffness factor, and sitosterol used as a replacement for diosgenin in some antihypertensive drugs, were prepared from soybeans. Isoflavones and phyto-oesterogens found in soybeans have been suggested to have a preventive effect against various cancers comprising breast, prostate, and colon cancers (Adlercreutz, H.; Phyto-oestrogens and cancer. The Lancet Oncology, 2002, Vol. 3, p. 364-373). Consumption of phytosterol-supplemented margarine was also found to lower total plasma cholesterol and LDL-cholesterol concentrations in older middle-aged hypercholesterolemic individuals (Matvienko, O. A., Lewis, D. S., Swanson, M., Aendt, B., Rainwater, D. L., Stewart, J., and Alekel, D. L.; A single daily dose of soybean phytosterols in ground beef decreases serum total cholesterol and LDL cholesterol in young, mildly hypercholesterolemic men. Am J Clin Nutr., 2002, 76, p. 57-64).

Some extracts from soybean have been also reported to have pharmaceutical effects. 1,1-diphenyl-2-picrylhydrazyl (DPPH) radical-scavenging activity of the 70 % aqueous acetone extract from the seed coat of the brown soybean variety, Akita-Zairai, was disclosed (Takahata. Y., O.-Kameyama, M., Furuta, S., Takahashi, M., and Suda, I.; Highly polymerized procyanidins in brown soybean seed coat with a high radical-scavenging activity. J. Agric. Food Chem., 2001, 49, p. 5843-5847). An extract from germ extracts, soybean, rice bran, tear grass, sesame, wheat, citron, green tea, green leaf extract, and malted rice, which were slowly roasted under a powdered oure at less than 60 °C and fermented with *Aspergillus oryzae* over 3 days to transform each ingredient into low molecular weight substances, was found to have antioxidative effects (Minamiyama, Y., Yoshikawa, T., Tanigawa, T., Takahashi, S., Naito, Y., Ichikawa, H., and Kondo, M.; Antioxidative effects of a processed grain food. J. Nutr. Sci. Vitaminol., 1994, 40, p. 467-477). Boiled extracts of green leaves of carrot, crucifers, and beans comprising black bean, red bean, mung bean, and soybean showed to have great anti-tumor-promoter and radical-scavenging activities (Maeda. H., Katsuki, T., Akaike, T. and Yasutake. R.; High correlation between lipid peroxide radical and tumor-promoter effect: suppression of tumor promotion in the Epstein-Barr virus/B-lymphocyte system and scavenging of alkyl peroxide radicals by various vegetable extracts. Jpn. J. Cancer Res., 1992, 83, p. 923-928). Water extract of black bean also reported to effect on acetaminophen-induced liver injury by measuring serum glutamate-oxalate-transaminase (sGOT) and serum glutamate-pyruvate-transaminase (sGPT) activities in rats (Wu, S.-J., Wang, J.-S. and Chang, C.-H.; Evaluation of hepatoprotective activity of legumes. Phytomedicine, 2001, Vol. 8(3), p. 213-219). An extract of soybean seed with an extracting liquid consisting of mixed system of low aliphatic alcohol and low boiling ester was shown as a refresh drink agent for quenching thirst (JP Patent No. 1117767).

Some specific extracts from soybean had been found to be applied in cosmetics or pharmaceuticals in treating some skin disease.

Soya extract, which contains sphingomyelins and phospholipids in defined ratios was disclosed to be used in cosmetics for the treatment of dry skin (U.S. Patent Pub. No. US2002/0009509 A1). Such extract was produced by extracting ripe whole soya beans or oil-free soya flour using aliphatic alcohols alone or in a mixture with water and followed by the treatment with aliphatic hydrocarbons and with aliphatic ketones. Therefore, the extract is liposoluble.

An acne medicine, comedon production inhibitor or cosmetic composition containing one or more plant extracts selected from whey, and a Phellodendeon amurense Ruprecht extract, and further one or more extracts selected from Scutellaria baicalensis Geoegi, Symphytum officinale Linne, and Glycine max (L.) Merrill, was found to be effective for preventing or treating skin diseases such as the acne or inflammatory chapped skin caused by the acne (JP Patent No. 2001097842). The composition can be used for production of general drinks, foods, and so on.

Malonylisoflavone glycoside obtained from soybean or an extracted solution of soybeans with water and, for example, malonyldaizin etc. was as an epithelial cell proliferation promoter (JP Patent No. 9059166). The glycoside is obtained by immersing peeled soybeans in water adjusted to pH 7.5 to pH 9.0 with caustic soda at 45-65 °C for 2-4 hours, removing the soybeans to give the immersion water as an extracted solution with water, removing protein from the extracted solution by an ultrafilter membrane to give a filtrate, bringing the filtrate into contact with an adsorbent and eluting the glycoside from the adsorbed material by using an aqueous solution of an alcohol or an alkali aqueous solution of an alcohol.

Products of fermenting soybean by microorganisms were also applied as anti-active oxygen action compositions, agents, foods, cosmetics and medicines (such as JP Patent No. 4139132).

However, none of the prior art teaches or suggests that a low concentration or free of alcohol vapor fraction from *Glycine max* (L.) Merr. which is prepared easily in treating skin injuries and dermatological disorders.

### SUMMARY OF THE INVENTION

The present invention provides a vapor fraction from the seeds of *Glycine max* (L.) Merr., which shows dramatic effects of treating skin injuries, treating dermatological disorders, stimulating cell regeneration, and stimulating hair growth.

The vapor fraction of the invention is prepared by the process comprising the steps of:
(a) providing a rude extract of the seeds in an alcohol solution containing alcohol at the concentration lower than about 15 % wt. or in water; and
(b) vaporizing the rude extract at a barometric pressure lower than about 1 atm and at a temperature lower than about 110 °C to obtain the vapor fraction.

The vapor fraction is different from any known seed extracts of *Glycine max* (L.) Merr. because its fingerprint shows a unique profile through a high performance liquid chromotography (HPLC). In particular, the HPLC spectrogram of the 50 µL of vapour fraction taken at 200 nm using Waters Sphersorb^{™} -ODS2 column with an inner diameter (I.D.) of 4.6 mm, a length (L) of 250 nm and a particle size of 5 µm comprises peaks at retention time of 2.910, 5.190, 13.190, and 50.815 minutes with the coefficient of variation of 8 %, where mobile phase at 0 to 5 minutes is 95% H₂O/5 CH₃CN; at 5 to 30 minutes is 95% H₂O/5 % CH₃CN in gradient, at 30 to 80. minutes is 75% H₂O/25 % CH₃CN in gradient; at 80 to 100 minutes is 0% H₂O/100 % CH₃CN in gradient; at 100 to 101 minutes is 0% H₂O/100 % CH₃CN in gradient; and at 101 minutes is 95% H₂O/5 % CH₃CN; and a flow rate is 1 mL/min.

The invention also provides a composition comprising the vapor fraction of the invention. In particular, the composition can be used for a pharmaceutical composition, a cosmetic composition or a skin cleaning composition.

According to the present invention, there is thus provided a vapor fraction from seeds of Glycine max (L.) Merr, which vapor fraction is obtainable by a process comprising:
(a) providing a crude extract of the seeds in an alcohol solution containing alcohol at a concentration lower than about 15% wt. or in water, and
(b) vaporizing the crude extract at a barometric pressure lower than about 1 atm and at a temperature lower than about 110°C to obtain the vapor fraction;
wherein a spectrogram of 50 µL of vapor fraction taken at 200 nm through a high performance liquid chromatography (HPLC) using Waters Sphersorb^{™}-ODS2 column with an inner diameter (I.D.) of 4.6 mm, a length (L) of 250 mm and a particle size of 5 µm comprises peaks at a retention time of 2.910, 5.190, 13.190, and 50.815 minutes with the coefficient of variation of 8%, where mobile phase at 0 to 5 minutes is 95% H₂0/5% CH₃CN; at 5 to 30 minutes is 95% H₂O/5% CH₃CN in gradient; at 30 to 80 minutes is 75% H₂O/25% CH₃CN in gradient; at 80 to 100 minutes is 0% H₂O/100% CH₃CN in gradient; at 100 to 101 minutes is 0% H₂O/100% CH₃CN in gradient; and at 101 minute is 95% H₂O/5% CH₃CN and the flow rate is 1 mL/min.

The invention also provides:
- a vapor fraction from the raw materials comprising seeds of Glycine max (L.) Merr and at least one of seeds of *Sesamum indicum,* seeds of *Phaseolus radiatus* L. or material from an antimicrobial herb, which vapor fraction is obtainable by a process comprising:
   (a) providing a crude extract of the raw materials in an alcohol solution containing alcohol at a concentration lower than about 15% wt. or in water, and
   (b) vaporizing the crude extract at a barometric pressure lower than about 1 atm and at a temperature lower than about 110°C to obtain the vapor fraction;
   wherein a spectrogram of 50 µL of vapor fraction taken at 200 nm through a high performance liquid chromatography (HPLC) using Waters Sphersorb^{™}-ODS2 column with an inner diameter (I.D.) of 4.6 mm, a length (L) of 250 mm and a particle size of 5 µm comprises peaks at a retention time of 2.910, 5.190, 13.190, and 50.815 minutes with the coefficient of variation of 8%, where mobile phase at 0 to 5 minutes is 95% H₂0/5% CH₃CN; at 5 to 30 minutes is 95% H₂O/5% CH₃CN in gradient; at 30 to 80 minutes is 75% H₂O/25% CH₃CN in gradient; at 80 to 100 minutes is 0% H₂O/100% CH₃CN in gradient; at 100 to 101 minutes is 0% H₂O/100% CH₃CN in gradient; and at 101 minute is 95% H₂O/5% CH₃CN and the flow rate is 1 mL/min;
- use of the vapor fraction of the invention for use in the manufacture of a medicament for use in treating skin injuries or dermatological disorders or for stimulating cell regeneration or stimulating hair growth;
- a composition comprising the vapor fraction of the invention;
- a process for the preparation of a vapor fraction from seeds of Glycine (L.) Merr, which process comprises:
   (a) providing a crude extract of the seeds in an alcohol solution containing alcohol at a concentration lower than about 15% wt. or in water; and
   (b) vaporizing the crude extract at a barometric pressure lower than about 1 atm and at a temperature lower than about 110°C to obtain the vapor fraction; and
- a process for the preparation of a vapor fraction from the raw materials comprising seeds of Glycine max (L.) Merr and at least one of seeds of *Sesamum indicum,* seeds of *Phaseolus radiatus* L. or a material from an antimicrobial herb, which process comprises:
   (a) providing a crude extract of the raw materials in an alcohol solution containing alcohol at a concentration lower than about 15% wt. or in water; and
   (b) vaporizing the crude extract at a barometric pressure lower than about 1 atm and at a temperature lower than about 110°C to obtain the vapor fraction.

### BRIEF DESCRIPTION OF THE DRAWINGS

FIG 1. illustrates the HPLC spectrogram of Fraction 1 obtained by the process according to the invention; FIG. 1a: 0 to 20 minutes; FIG. 1b: 20 to 40 minutes; FIG. 1c: 40 to 60 minutes.
FIG 2. illustrates the HPLC spectrogram of Fraction 3 obtained by the process according to the invention; FIG. 2a: 0 to 20 minutes; FIG. 2b: 20 to 40 minutes; FIG. 2c: 40 to 60 minutes.
FIG. 3 illustrates the picture of full thickness skin wounds treated with the ointment according to the invention in Example 3.
FIG. 4 illustrates the picture of hair growth on full thickness skin wounds treated with the ointment according to the invention in Example 3; FIG. 4a: the ointments comprising Fractions 1 to 3; FIG. 4b: the ointments comprising Fractions 4 to 6.
FIG. 5 illustrates the result of histological examination of deep partial thickness burn wounds without treatment in Example 5.
FIG. 6 illustrates the result of histological examination of deep partial thickness burn wounds treated with silver sulfadiazine drug in Example 5.
FIG. 7 illustrates the result of histological examination of deep partial thickness burn wounds treated with the ointment according to the invention in Example 5.
FIG. 8 illustrates the result of histological examination of large area full thickness skin wounds treated with silver sulfadiazine drug in Example 6.
FIG. 9 illustrates the result of histological examination of large area full thickness skin wounds treated with the ointment according to the invention in Example 6.
FIG. 10 illustrates the result of histological examination of irradiation skin injury treated with the ointment according to the invention in Example 7.
FIG. 11 illustrates the result of histological examination of irradiation skin injury without treatment in Example 7.
FIG. 12 illustrates the result of normal tissue in Example 7.
FIG. 13 illustrates the picture of dermatitis on rabbit ear before treatment in Example 8.
FIG. 14 illustrates the picture of dermatitis on rabbit ear after treated with the ointment according to the invention on Day 1 in Example 8.
FIG. 15 illustrates the picture of dermatitis on rabbit ear after treated with the ointment according to the invention on Day 2 in Example 8.
FIG. 16 illustrates the picture of dermatitis on rabbit ear after treated with the ointment according to the invention on Day 3 in Example 8.
FIG. 17 illustrates the picture of diabetes mellitus wounds in Example 9.
FIG. 18 illustrates the picture of diabetes mellitus wounds in Example 9.
FIG. 19 illustrates the picture of diabetes mellitus wounds treated with the ointment according to the invention for 2 months in Example 9.
FIG. 20 illustrates the picture of diabetes mellitus wounds treated with the ointment according to the invention for 5 months in Example 9.
FIG. 21 illustrates the picture of diabetes mellitus wounds treated with the ointment according to the invention for 5 months in Example 9.
FIG. 22 illustrates the picture of chronic stasis ulcer in Example 10.
FIG. 23 illustrates the picture of chronic stasis ulcer treated with the ointment according to the invention for 2 weeks in Example 10.
FIG. 24 illustrates the picture of chronic stasis ulcer treated with the ointment according to the invention for 4 weeks in Example 10.
FIG. 25 illustrates the picture of ecchymosis in Example 11.
FIG. 26 illustrates the picture of ecchymosis treated with the ointment according to the invention for 3 days in Example 11.
FIG. 27 illustrates the picture of ecchymosis treated with the ointment according to the invention for 7 days in Example 11.
FIG. 28 illustrates the picture of abrasion wounds in Example 12.
FIG. 29 illustrates the picture of ecchymosis treated with the ointment according to the invention for 7 days in Example 12.
FIG. 30 illustrates the picture of suture wounds treated with the ointment according to the invention in Example 13.
FIG. 31 illustrates the picture of atopic dermatitis in Example 14.
FIG. 32 illustrates the picture of atopic dermatitis treated with the ointment according to the invention for 1 week in Example 14.
FIG. 33 illustrates the picture of atopic dermatitis treated with the ointment according to the invention for 4 weeks in Example 14.
FIG. 34 illustrates the picture of atopic dermatitis treated with the ointment according to the invention for 6 weeks in Example 14.

### DETAILED DESCRIPTION OF THE INVENTION

According to the invention, the vapor fraction of *Glycine max* (L.) Merr. is unexpectedly found to have dramatic effects in treating skin injuries, treating dermatological disorders, stimulating cell regeneration, and stimulating hair growth.

According to the present invention, a vapor faction from seeds of *Glycine max* (L.) Merr. is prepared by the process comprising the steps of:
(a) providing a crude extract of the seeds in an alcohol solution containing alcohol at the concentration lower than about 15 % wt. or in water; and
(b) vaporizing the crude extract at a barometric pressure lower than about 1 atm and at a temperature lower than about 110 °C to obtain the vapor fraction.

It is unexpectedly found that a vapor fraction obtained from a crude extract of the seeds in an alcohol solution containing alcohol at the concentration lower than about 15 % wt. or in water has effect in dermatology. The vapor fractions are easily prepared. Medicinal or cosmetic products prepared from seeds of *Glycine max* (L.) Merr. are advantageous because (1) soybeans have been consumed for a long time and are believed to be non-toxic; and (2) soybeans are not expensive.

In an embodiment of the invention, the crude extract from the seeds of *Glycine max* (L.) Merr. is obtained by an extraction with an alcohol solution at a concentration lower than about 15 % wt, or with water. Any methods for obtaining rude extracts from plant seeds commonly used in the art can be used to practice the invention. For instance, the rude extract can be obtained by dividing the seeds into small pieces in any conventional manners such as grinding, stirring, disturbing, cutting or mincing, and immersing them in an alcohol solution at a concentration lower than about 15 % wt. In a preferred embodiment, the concentration of alcohol is lower than about 10 % wt, more preferably about 3 % wt or about 2 % wt. According to the invention, the crude extract can also be extracted with water. In a preferred embodiment, the pieces of seeds are immersed in alcohol or water ranging from about 5 parts to about 10 parts by weight. Any ways of dividing the seeds and extracting the divided seeds commonly used in the art can be used to practice the present invention.

According to the invention, the crude extract is vaporized at a barometric pressure lower than about 1 atm and at a temperature lower than about 110 °C, preferably from about 60 °C to about 110 °C. The vapor fraction can be collected in a liquid form by chilling the vapor.

In a preferred embodiment of the invention, a process of vaporizing the crude extract at a given barometric pressure and temperature, and collecting said vapor fraction by chilling the vapor can be performed in a rotary evaporator where the vapor is evaporated to the condensing tube supplied with cold water, and then the vapor is chilled by passing through the condensing tube to collect the vapor fraction in a liquid form. The manipulation is simple and the cost is low.

The vapor fraction according to the invention can be analyzed and identified with a high performance liquid chromatography (HPLC). For instance, the HPLC spectrogram of the 50 µL of vapor fraction taken at 200 nm using Waters Sphersorb^{™}-ODS2 column with an inner diameter (I.D.) of 4.6 mm, a length (L) of 250 mm and a particle size of 5 µm comprises peaks at retention time of 2.910, 5.190, 13.190, and 50.815 minutes with the coefficient of variation of 8 %, where mobile phase at 0 to 5 minutes is 95 % H₂O/5 % CH₃CN; at 5 to 30 minutes is 95 % H₂O/5 % CH₃CN in gradient, at 30 to 80 minutes is 75 % H₂O/25 % CH₃CN in gradient; at 80 to 100 minutes is 0 % H₂O/100 % CH₃CN in gradient; at 100 to 101 minutes is 0 % H₂O/100 % CH₃CN in gradient; and at 101 minute is 95 % H₂O/5 % CH₃CN; and a flow rate is 1 mL/min. According to the spectrogram, substances in the vapor fraction show strong polarity. They are much different from other ingredients disclosed in the prior art. For example, daidzein and genistein have four rings in their structures, and have weak polarity.

In one preferred embodiment of the invention, several herbals are added for assisting seeds of *Glycine max* (L.) Merr. in treating skin injuries, treating dermatological disorders, stimulating cell regeneration, and stimulating hair growth. According to the invention, a vapor fraction is obtained from raw materials comprising seeds of *Glycine max* (L.) Merr. and at least one material selected from the group consisting of seeds of *Sesamum indicum,* seeds of *Phaseolus radiatus* L. and antimicrobial herbals. The preparation of vapor fraction from the raw material and the analysis and identification of the vapor fraction are described above. Preferably, the antimicrobial herbal is selected from the group consisting of dry rhizomes *Rheum palmatum* L., dry rhizomes of Rhei Rhizoma, dry barks of *Phellodendron amurense Rupr.,* and dry barks of Phellodendri Cortex. In another aspect, the raw materials preferably comprise about 40 to about 100 % of seeds of *Glycine max* (L.) Merr., 0 to about 50 % of seeds of *Sesamum indicum*, 0 to about 50 % of seeds of *Phaseolus radiatus* L. and 0 to about 30 % of antimicrobial herbal.

In one more preferred embodiment of the invention, the raw materials comprise about 70 % of seeds of *Glycine max* (L.) Merr., about 20 % of seeds of *Sesamum indicum*, about 5 % of dry rhizomes of *Rheum palmatum* L. or dry rhizomes of Rhei Rhizoma, and about 5 % of dry barks of *Phellodendron amurense Rupr.* or dry barks of Phellodendri Cortex. The raw material is in about 2 % of alcohol.

In another more preferred embodiment of the invention, the raw materials comprise about 80 % of seeds of *Glycine max* (L.) Merr. and about 20 % of seeds of *Phaseolus radiatus* L. The raw material is in about 2 % of alcohol.

In still another more preferred embodiment of the invention, the raw materials comprise about 80 % of seeds of *Glycine max* (L.) Merr. and about 20 % of seeds of *Sesamum indicum.* The raw material is in about 2 % of alcohol.

In still another more preferred embodiment of the invention, the raw materials comprise about 90 % of seeds of *Glycine max* (L.) Merr. and about 10 % of dry rhizomes of *Rheum palmatum* L. or dry rhizomes of Rhei Rhizoma. The raw material is in water.

In still another more preferred embodiment of the invention, the raw materials comprise about 80 % of seeds of *Glycine max* (L.) Merr., about 10 % of seeds of *Sesamum indicum* and about 10 % of dry rhizomes of *Rheum palmatum* L. or dry rhizomes of Rhei Rhizoma. The raw material is in water.

The invention also provides a composition comprising the vapor fraction according to the invention. Preferably, the composition is selected from the group consisting of a pharmaceutical composition, a cosmetic composition and a skin cleaning composition. In a preferred embodiment according to the invention, the composition further comprises an antimicrobial agent such as borneol. Preferably, the amount of the vapor fraction according to the invention ranges from about 70 % to about 80 % of total composition weight.

In one embodiment of the invention, the composition further comprises borneol, stearic acid, stearyl alcohol, palmitic acid, cetyl alcohol, beewax and polyoxy ethylene sorbitan monostearate (POE) to form an ointment for easily applied on a subject.

A method for treating skin injuries or dermatological disorders comprising applying the composition according to the invention at a sufficient amount effective for treating skin injuries, treating dermatological disorders, stimulating cell regeneration, and stimulating hair growth.

The vapor fraction according to the invention is useful for treating skin injuries, treating dermatological disorders, stimulating cell regeneration, and stimulating hair growth. For example, the skin injuries or dermatological disorders contains burn injury, sunburns, irradiation injury, acute and chronic trauma, ecchymosis, dermatitis, macule, papule, nodule, vesicle, bulla, pustule, wheal, plaque, cyst, scales, crust, fissure, ulcer, acne, xeroderma, desquamation, itch, allergic dermatitis, exanthema, and hair follicle defect. Preferably, the composition according to the invention is effective in treating burn injury, irradiation injury, acute and chronic trauma, dermatitis, gangrene, chronic stasis ulcer, abrasion, atopic dermatitis, suture wounds, diabetes mellitus wounds, and hair follicle defect.

In the animal model described below, the vapor fraction according to the invention has been proved to be effective for curing large area skin deficient without granulation, epulosis and allergy. In addition, hair follicle defect could be cured and hairs could grow again without erythema or eschar. The healing rates and conditions of the wounds treated with the vapor fraction according to the present invention are much better than those with conventional drugs. The wounds treated with the vapor fraction according to the present invention were healed without scar, and the skin color of the healed wound was the same as normal skin color. Besides, in the histological examination, the skin tissues of the wounds treated with the vapor fraction according to the present invention did not show fibrous degeneration that usually caused enormous damage on appearance and produced complex reconstructive surgenes.

In addition, no allergic reaction, inflammation, blain, or rash were found in the wounds treated with the vapor fraction of the invention. After the treatment, the accessory organs and cells of the wound grew as the same as those of normal skins. By contrary, the wounds treated with a conventional drug have less numbers of accessory organs and cells, and the fiber width of the wounds was larger than the normal.

Furthermore, the vapor fraction according to the invention is effective in treating irradiation injuries. So far, no drugs are effective in treatment of irradiation injuries. It is unexpectedly found that in a subject suffering from irradiation injuries, the wounds after the treatment with the vapor fraction show little damage in hairs and no allergy.

In addition, neither inflammation nor tissue contraction, which are usually observed in wound healing, is found in the wounds healed with the vapor fraction according to the invention. The vapor fraction is effective in healing the wounds in which the dermal is damaged. Therefore, the invention provides a better, convenient, and economical way for treating large area and dermatological wounds.

The following Examples are given for the purpose of illustration only.

### Example 1: Preparation of Vapor Fraction from Seeds of Glycine max (L.) Merr.

Raw materials comprising seeds of *Glycine max* (L.) Merr., seeds of *Sesamum indicum,* seeds of *Phaseolus radiatus* L., dry rhizomes of *Rheum palmatum* L. or dry rhizomes of Rhei Rhizoma, and dry barks of *Phellodendron amurense Rupr.* or dry barks of Phellodendri Cortex were ground and extracted with 8 parts by weight of alcohol or distilled water to obtain a rude extract. The contents were shown in Table 1. The vapor fraction was obtained by vaporizing the rude extract in a rotary evaporator (EYELA N-1000S, 1000S-W) at a pressure of lower than 1 atm and a temperature of 60 °C, and passing through a condensing tube supplied with cold water.

**Table 1:**

| Raw materials | Fraction No: | | | | | | | |
|---|---|---|---|---|---|---|---|---|
| | 1 | 2 | 3 | 4 | 5 | 6 | 7 | 8 |
| Seeds of *Glycine max* (L.) Merr. (%) | 100 | 70 | 53 | 80 | 0 | 80 | 90 | 80 |
| Seeds of *Sesamum indicum* (%) | 0 | 20 | 20 | 0 | 0 | 20 | 0 | 10 |
| Seeds of *Phaseolus radiatus* L*.* (%) | 0 | 0 | 17 | 20 | 0 | 0 | 0 | 0 |
| dry rhizomes of *Rheum palmatum* L. or dry rhizomes of Rhei Rhizoma (%) | 0 | 5 | 5 | 0 | 0 | 0 | 10 | 10 |
| dry barks of *Phellodendron amurense Rupr.* or dry barks of Phellodendri Cortex (%) | 0 | 5 | 5 | 0 | 0 | 0 | 0 | 0 |
| Solution | | | | | | | | |
| Distilled water (%) | 98 | 98 | 98 | 98 | 100 | 98 | 100 | 100 |
| Ethanol (%) | 2 | 2 | 2 | 2 | 0 | 2 | 0 | 0 |

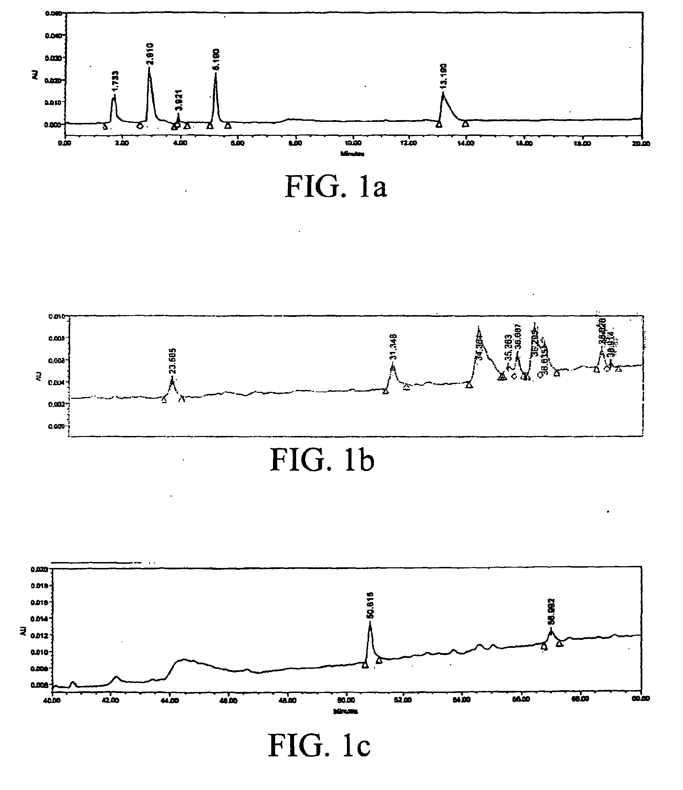

The result of the HPLC for 50 µL of Fraction 1 was shown in FIG. 1. The HPLC was preformed through Waters Sphersorb^{™}-ODS2 column with an inner diameter (I.D.) of 4.6 mm, a length (L) of 250 mm and a particle size of 5 µm where the mobile phase at 0 to 5 minutes was 95 % H₂O/5 % CH₃CN; at 5 to 30 minutes was 95 % H₂O/5 % CH₃CN in gradient, at 30 to 80 minutes was 75 % H₂O/25 % CH₃CN in gradient; at 80 to 100 minutes was 0 % H₂O/100 % CH₃CN in gradient; at 100 to 101 minutes was 0 % H₂O/100 % CH₃CN in gradient; and at 101 minute was 95 % H₂O/5 % CH₃CN and the flow rate was 1 mL/min. The HPLC was conducted with Waters Alliance 2795 HT, and the spectrogram was taken from 190 to 400 nm by Waters Photo Diode Array 2996. As shown in the spectrogram taken at 200 nm, there were sharp peaks at the retention time of 2.910, 5.190, 13.190, and 50.815 minutes with the coefficient of variation of 8 %.

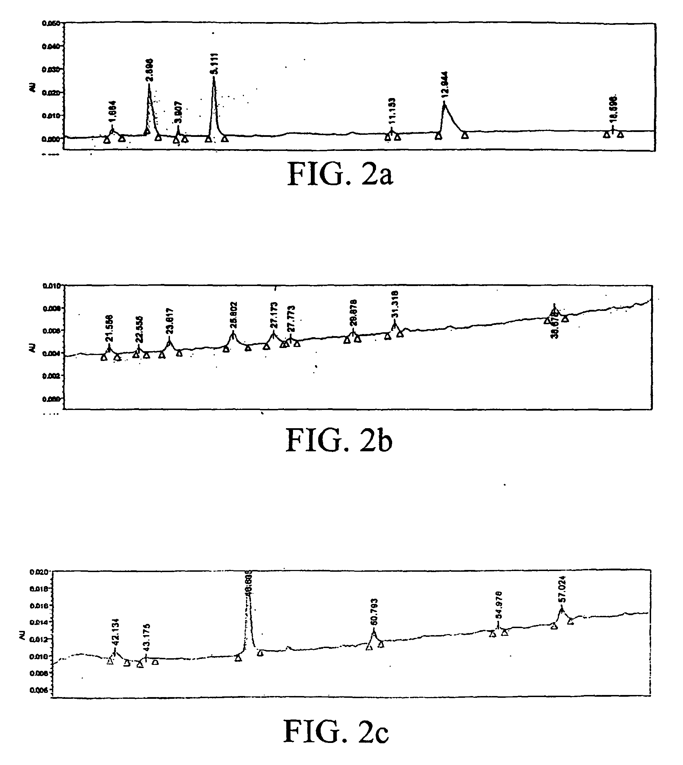

The result of the HPLC for 50 µL of Fraction 3 was shown in FIG. 2. The HPLC was preformed through Waters Sphersorb^{™}-ODS2 column with an inner diameter (I.D.) of 4.6 mm, a length (L) of 250 mm and a particle size of 5 µm where the mobile phase at 0 to 5 minutes was 95 % H₂O/5 % CH₃CN; at 5 to 30 minutes was 95 % H₂O/5 % CH₃CN in gradient, at 30 to 80 minutes was 75 % H₂O/25 % CH₃CN in gradient; at 80 to 100 minutes was 0 % H₂O/100 % CH₃CN in gradient; at 100 to 101 minutes was 0 % H₂O/100 % CH₃CN in gradient; and at 101 minute was 95 % H₂O/5 % CH₃CN and the flow rate was 1 mL/min. The HPLC was conducted with Waters Alliance 2795 HT, and the spectrogram was taken from 190 to 400 nm by Waters Photo Diode Array 2996. As shown in the spectrogram taken at 200 nm, there were sharp peaks at the retention time of 2.896, 5.111, 12.944, and 46.603 minutes with the coefficient of variation of 8 %.

### Example 2: Preparation of Ointment

The ointments according to the invention were exemplified in Table 2:

**Table 2:**

| | Content (wt) |
|---|---|
| Fraction | 77 % |
| Borneol | 0.6 % |
| Stearic acid | 5 % |
| Stearyl alcohol | 4 % |
| Palmitic acid | 4% |
| Cetyl alcohol | 5% |
| Beewax | 2 % |
| Polyoxy ethylene sorbitan monostearate | 2.4 % |

The ointments of the invention can be prepared in any conventional methods commonly used in this art.

### Example 3: Effects on Full Thickness Skin Wounds in Rabbits

### Treatment of Animals :

Five female adult New Zealand White rabbits weighting 2.9 to 3.1 Kg was taken as an animal model. Each rabbit was caged alone and feed with chow and water. The rabbits were anaesthetized with 25 to 40 mg/Kg of ketamine HCl. The back of each rabbit was thoroughly clipped and six areas of the full thickness skin (wherein each area is of 1.5 x 1.5 cm²) were excised, wherein the full thickness skin comprised epidermis, dermis, and panniculus carnosus.

### Dosage and Frequency:

The six areas of the wound of each rabbits were treated twice a day with the ointments comprising Fractions 1 to 6, respectively. The ointments were topically applied on the wounds at an amount of 0.35 g per cm².

### Clinical Wound Evaluation:

The wounds were observed everyday and photographed with a referential scale three times a week under a uniform condition of light, a focal length, and aperture to record the process of the wound healing. Besides, the sizes of the wounds were also measured and given in Table 3.

**Table 3:**

| Day | Wound area treated with the ointment comprising Fraction No. (%) | | | | | |
|---|---|---|---|---|---|---|
| | 1 | 2 | 3 | 4 | 5 | 6 |
| 0 | 100.0 | 100.0 | 100.0 | 100.0 | 100.0 | 100.0 |
| 2 | 105.5 | 103.1 | 103.8 | 105.1 | 120.9 | 117.3 |
| 5 | 108.9 | 110.2 | 105.5 | 100.4 | 134.4 | 130.0 |
| 8 | 80.0 | 72.9 | 78.3 | 75.3 | 93.0 | 96.0 |
| 11 | 49.4 | 47.1 | 49.4 | 39.2 | 54.0 | 55.1 |
| 14 | 17.9 | 15.1 | 17.0 | 16.5 | 21.0 | 20.4 |
| 17 | 7.7 | 6.2 | 9.4 | 9.1 | 10.2 | 10.7 |
| 19 | 3.6 | 4.1 | 4.1 | 4.4 | 7.4 | 5.0 |
| 22 | 0.5 | 0.5 | 0.0 | 0.5 | 1.7 | 0.5 |
| 25 | 0.0 | 0.0 | 0.0 | 0.0 | 0.0 | 0.0 |

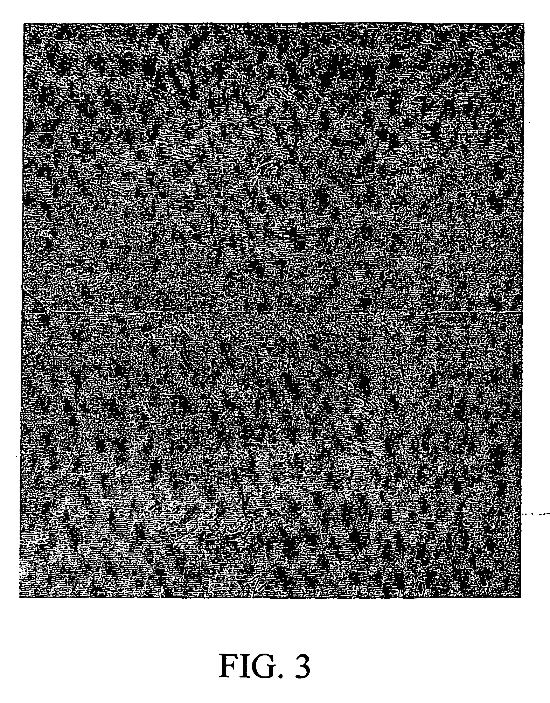

The result show that the wounds were all healed (as shown in FIG. 3). The excipient according to the invention (ointment comprising Fraction 5) also had an effect in treating full thickness skin wounds.

### Hair Growth Evaluation:

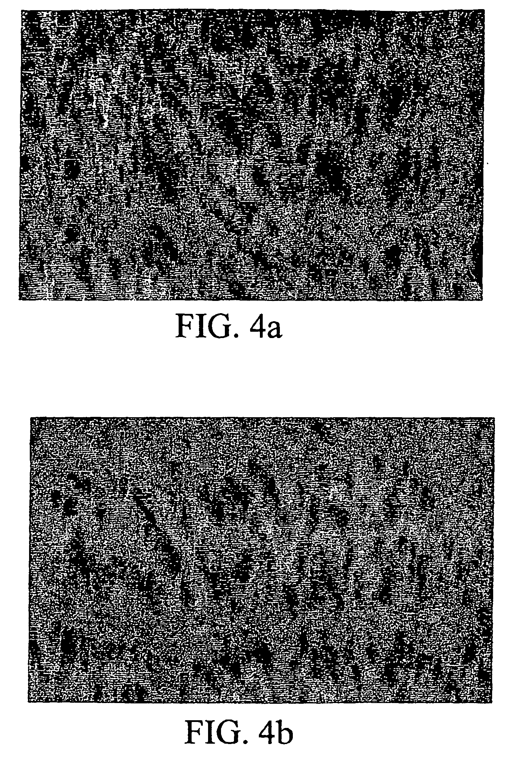

The length of new hair was measured. Hairs of the all wounds treated with the ointments comprising Fractions 1 to 6 were of about 1.76 cm long in average (as shown in FIG. 4), and those of the areas without treatment were average 0.1 to 0.2 cm.

The result showed that the vapor fraction according to the invention had an ability to repair hair follicle defect and stimulated hair growth. Besides, the excipient according to the invention was effective to enhance hair growth, too.

### Example 4: Effects on Full Thickness Skin Wounds in Rats

### Treatment of Animals :

The male adult Sprague-Dawley rats were purchased from Laboratory Animal Center of the National Cheng Kung University in Taiwan. The rats weighting 300 to 350g were anaesthetized with 25 to 40 mg/Kg of ketamine HCl. The back of each rat was thoroughly clipped using an electrical clipper. Each rat was caged alone and feed with chow and water. For each rat, the full thickness skin of an area of 2.5 x 2.5 cm² was excised, wherein the full thickness skin comprises epidermis, dermis, and panniculus carnosus (B. Hafemann et al. Use of a collagen/elastin-membrane for the tissue engineering of dermis. Burns. 1999;25:373-384; H.-J. Wang et al. Use of a Porcine Dermis Template to Enhance Widely Expanded Mesh Autologus Split-Thickness Skin Graft Growth. J Trauma. 1997 Feb;42(2): 177-182).

### Dosage and Frequency:

Two-experiment group of rats were treated with the ointments comprising Fractions 7 and 8 as given in Example 2 and each group contained three rats. The pharmaceutical compositions were topically applied on the wounds at an amount of 0.35 g per cm² and 0.1 to 0.2 cm height. All the wounds were dressed by sterile gauze and elastic bandage, and the dressing was changed everyday.

The evaluation methods analogous to those in Example 3 were used and the sizes of the wounds were measured and given in Table 4.

**Table 4:**

| Day | Wound area treated with the ointment comprising Fraction No. (%) | |
|---|---|---|
| | 7 | 8 |
| 0 | 100 | 100 |
| 2 | 85.3 | 102.5 |
| 5 | 64.3 | 71.4 |
| 9 | 24 | 34.5 |
| 12 | 18.6 | 25.2 |
| 15 | 9.3 | 13.4 |
| 17 | 3.1 | 8.4 |
| 20 | 0.4 | 2.5 |
| 23 | 0.0 | 0.0 |

The wounds treated with the ointments comprising Fractions 7 and 8 were healed. Particularly, the wound area treated with the pharmaceutical composition comprising Fraction 7 decreased more quickly.

### Example 5: Effects on Healing of Deep Partial Skin Thickness Burn Wounds

### Treatment of Animals:

The female adult Sprague-Dawley rats were purchased from Laboratory Animal Center of the National Cheng Kung University in Taiwan. Nine rats weighting 230 to 280 g were anaesthetized with 25 to 40 mg/Kg of ketamine HCl. The back of each rat was thoroughly clipped using an electrical clipper at a predicted wound area in the middle of the back. Each rat was caged alone and feed with chow and water. Burn wounds of the rats were produced by using the method as described by Walker and Mason (Z-R. Gao et al. Porcine dermal collagen as a wound dressing for skin donor sites and deep partial skin thickness burns. Burns. 1992;18(6):492-496), in which the dorsal skin surface was exposed to water at 75 °C for 15 seconds through a template designed to produce a deep partial skin thickness burn with the area of 4 x 4 cm².

### Dosage and Frequency:

The experiment group (G3) of rats was treated with Pharmaceutical composition according to the invention that comprises Fraction 9 similar to Fraction 3 except the content of alcohol being 3 % as given in Example 2. There were one control group (G1) of rats without treatment and one comparative group (G2) of rats treated with a silver sulfadiazine drug (SWISS PHARMACEUTICAL CO., LTD.). Each group contained three rats. The drugs were topically applied on the wounds at an amount of 0.35 g per cm² twice a day until hairs grew up.

### Clinical Wound Evaluation:

The evaluation methods analogous to those in Example 3 were used and the sizes of the wounds were measured and given in Table 5.

**Table 5:**

| Day | G1 | | G2 | | G3 | |
|---|---|---|---|---|---|---|
| | Wound area (mm²) | Change of initial area (%) | Wound area (mm²) | Change of initial area (%) | Wound area (mm²) | Change of initial area (%) |
| 1 | 1260.00 | 100.00 | 1140.67 | 100.00 | 1776.33 | 100.00 |
| 5 | 938.67 | 74.50 | 1053.33 | 92.34 | 744.00 | 41.88 |
| 11 | 285.33 | 22.65 | 428.00 | 37.52 | 154.67 | 8.71 |
| 14 | 188.00 | 14.92 | 234.67 | 20.57 | 34.67 | 1.95 |
| 18 | 0.00 | 0.00 | 141.33 | 12.39 | 0.00 | 0.00 |
| 24 | 0.00 | 0.00 | 64.67 | 5.67 | 0.00 | 0.00 |
| 31 | 0.00 | 0.00 | 41.33 | 3.62 | 0.00 | 0.00 |
| 37 | 0.00 | 0.00 | 32.37 | 2.86 | 0.00 | 0.00 |

In the progress of the wound healing, the wounds treated with the ointment according to the invention were cured without scar and the skin color was as the same as the normal. However, scars were formed on the wounds treated with silver sulfadiazine drug. In addition, the wounds of the rats were cured on Day 18 faster than those with a conventional silver sulfadiazine drug that remained 2.86 % of area of wound on Day 37.

New hair growth scores were also estimated and recorded. The new hair growth scores were defined as follows: "-2" referring to no new hair growth with some eschar and erythema; "-1" referring to no new hair growth with some erythema; "0" referring to no new hair growth with normal skin; "+1" referring to some new hair growth; and "+2" referring to new hair growth of approximately 50% of the wound area.

The new hair growth scores were listed in Table 6:

**Table 6**

| Day | Score | | |
|---|---|---|---|
| | G1 | G2 | G3 |
| 15 | -2 | -2 | 0 |
| 18 | -2 | -2 | 1 |
| 21 | -1 | -1 | 1 |
| 24 | -1 | -1 | 1 |
| 27 | -1 | -1 | 1 |
| 30 | -1 | -1 | 1 |
| 33 | -1 | -1 | 1 |
| 35 | -1 | -1 | 1 |

As shown in Table 6, the ointment according to the invention was more effective in new hair growth than the conventional silver sulfadiazine drug. New hair growth was observed when treating Pharmaceutical composition according to the invention on Day 18, and 50 % of new hair growth was observed on Day 35. Neither erythema nor eschar was found in the wounds treated with Pharmaceutical composition according to the invention. However, not hair growth was observed when treating the conventional drug.

### Histological Evaluation of Skin:

After the wounds were completely healed, the skin tissues of each rat were taken and anaesthetized for histological examination using the evaluation methods as described by B. Hafemann *et al*. (B. Hafemann et al. Use of a collagen/elastin-membrane for the tissue engineering of dermis. Burns. 1999;25:373-384.). The samples were taken and stored in formaldehyde. Each specimen was embedded in a paraffin block, and thin sections were prepared, and stained by using the hematoxylin-eosin method. The specimens were observed under an optical microscope, and a transmission electron microscope.

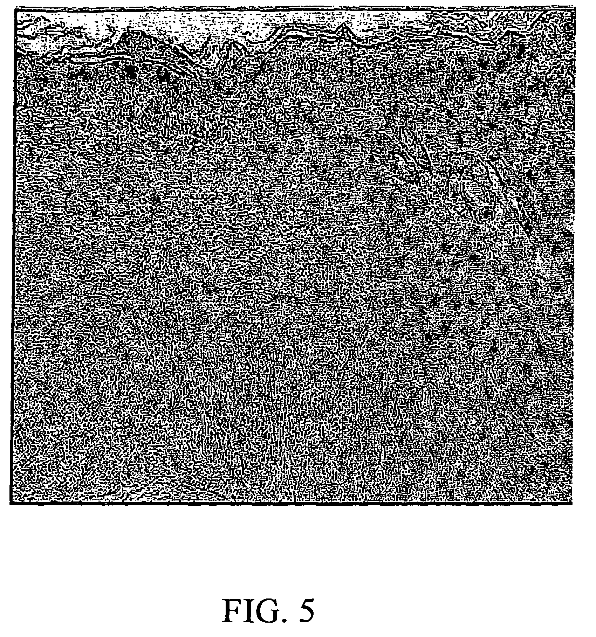

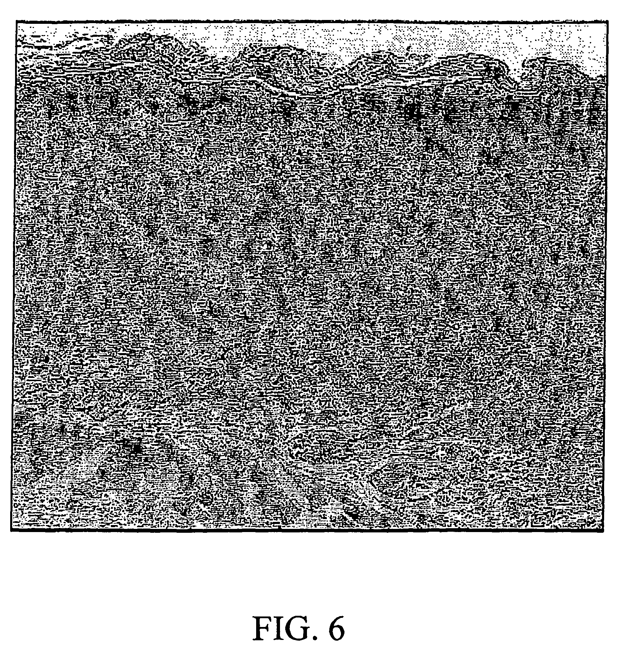

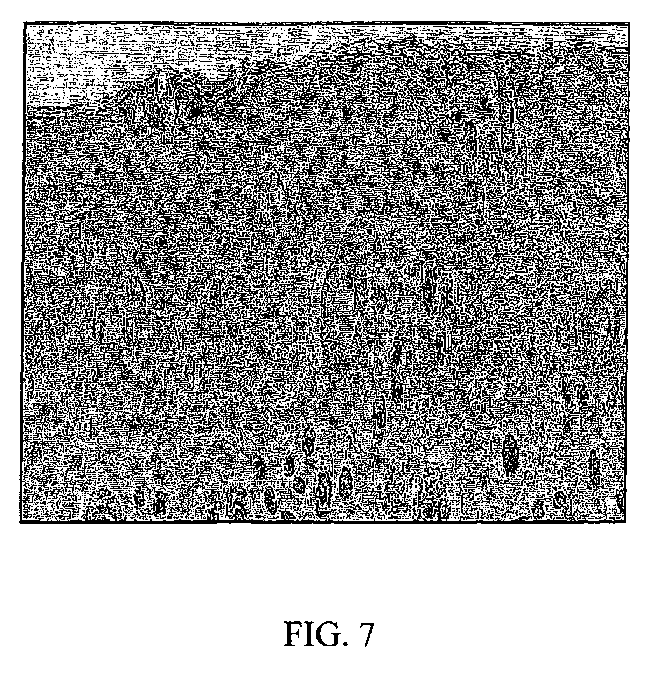

The results of the histological examination showed that the thickness of epithelium tissues of the rats without treatment (as shown in FIG. 5) was not even, and the granular layer was thickened. The fiber layer was observed in the deep dermal. In the group of rats treated with the conventional silver sulfadiazine drug (as shown in FIG. 6) were also shown to have uneven thickness of epithelium tissues. The fiber layer was observed in the deep dermal, and the fair follicles were destroyed. On the other hand, the wounds (as shown in FIG. 7) treated with the ointment according to the invention appeared to be normal, and a large number of hair follicles were regenerated. The fiber layer was observed in the surface area of dermal.

### Example 6: Effects on Large Area Full Thickness Skin Wounds in Rats

### Treatment of Animals :

The male adult Sprague-Dawley rats were purchased from Laboratory Animal Center of the National Cheng Kung University in Taiwan. The rats weighting 300 to 350g were anaesthetized with 25 to 40 mg/Kg of ketamine HCl. The back of each rat was thoroughly clipped using an electrical clipper. Each rat was caged alone and feed with chow and water. For each rat, the full thickness skin at an area of 4.0 x 4.0 cm² was excised, wherein the full thickness skin comprises epidermis, dermis, and panniculus carnosus.

### Dosage and Frequency:

The experiment group of rats was treated with the ointment comprising Fraction 9 as given in Example 5. One comparative group (G2) of rats treated with a silver sulfadiazine drug (SWISS PHARMACEUTICAL CO., LTD.) was also provided. Each group contained five rats. The ointments were topically applied on the wounds at an amount of 0.75 g per cm² and 0.1 to 0.2 cm height. All the wounds were dressed by sterile gauze and elastic bandage, and the dressing was changed everyday.

The evaluation methods analogous to those in Example 3 were used and the sizes of the wounds were measured and given in Table 7.

**Table 7:**

| Day | silver sulfadiazine drug | | Ointment comprising Fraction 3 | |
|---|---|---|---|---|
| | Wound area (mm²) | Change of initial area (%) | Wound area (mm²) | Change of initial area (%) |
| 1 | 1977.60 | 100.00 | 1957.60 | 100.00 |
| 3 | 1763.20 | 89.16 | 1784.80 | 91.17 |
| 5 | 1444.80 | 73.06 | 1506.40 | 76.95 |
| 7 | 990.40 | 50.08 | 1265.60 | 64.65 |
| 9 | 556.00 | 28.11 | 768.80 | 39.27 |
| 11 | 429.60 | 21.72 | 456.80 | 23.33 |
| 13 | 368.80 | 18.65 | 385.60 | 19.70 |
| 15 | 283.20 | 14.32 | 299.20 | 15.28 |
| 17 | 208.80 | 10.56 | 236.80 | 12.10 |
| 19 | 166.00 | 8.39 | 238.40 | 12.18 |
| 21 | 131.00 | 6.62 | 161.60 | 8.26 |
| 23 | 119.00 | 6.02 | 123.20 | 6.29 |
| 25 | 87.00 | 4.40 | 88.00 | 4.50 |
| 27 | 45.00 | 2.28 | 76.00 | 3.88 |
| 29 | 40.00 | 2.02 | 56.00 | 2.86 |
| 31 | 35.00 | 1.77 | 34.40 | 1.76 |
| 35 | 18.00 | 0.91 | 20.00 | 1.02 |
| 33 | 16.00 | 0.81 | 8.00 | 0.41 |
| 37 | 0.00 | 0.00 | 0.00 | 0.00 |

In the progress of the wound healing, the wounds treated with the ointment according to the invention were cured without allergy. However, scars were formed on the wounds treated with silver sulfadiazine drug. In addition, severe allergy was observed in the wounds treated with silver sulfadiazine drug. The healing rate of the rats treated with the pharmaceutical composition according to the invention (about 25 to 31 days) was faster than that with the silver sulfadiazine drug (about 37 days).

### Histological Evaluation of Skin:

The method for histological evaluation was as described in Example 5.

The results of the histological examination taken on Day 45 showed that the wounds treated with the conventional silver sulfadiazine drug were observed to have the fiber layer in the deep dermal. Some epithelium cells died and form the callus. Chronic inflammation and eosinophils infiltration were both observed in the dermal. Some fiber also formed 0.5 cm away from the wound.

In the group of rats treated with the ointment according to the invention, the newborn epithelium was even, and a large amount of hair follicles were generated. Only little fiber formed.

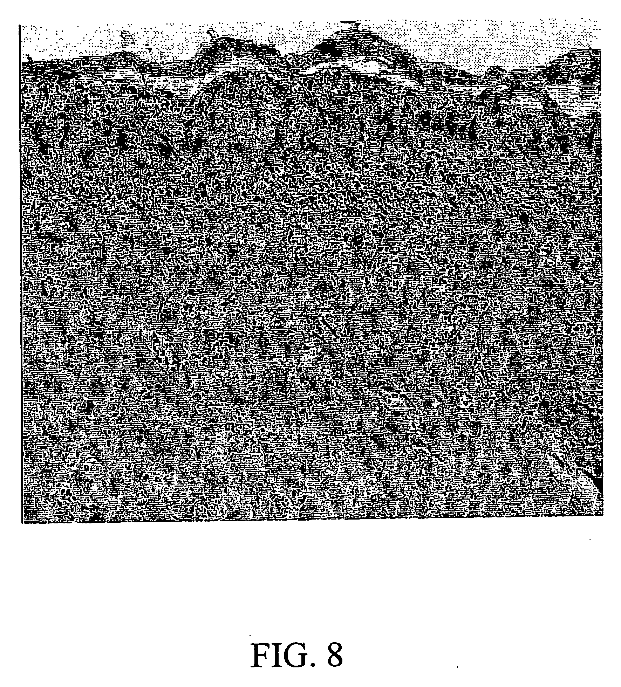

The results of the histological examination taken on Day 95 showed that the wounds treated with the conventional silver sulfadiazine drug were observed to have the uneven epithelium layer (as shown in FIG. 8). Fiber layer was form in the deep dermal. A small amount of hair follicles was generated.

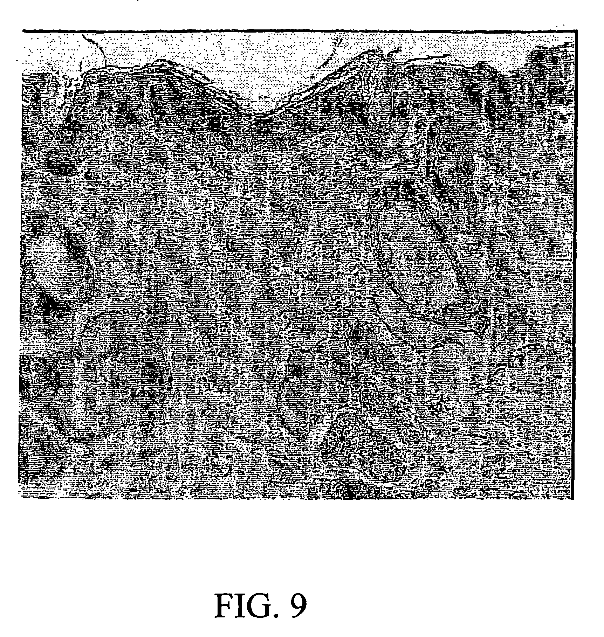

In the group of rats treated with the ointment according to the invention, the newborn epithelium was even, and a large amount of hair follicles were generated (as shown in FIG. 9).

### Example 7: Effects on Local Electron Irradiation Skin Injuries

### Treatment of Animals:

The female adult Sprague-Dawley rats were purchased from Laboratory Animal Center of the National Cheng Kung University in Taiwan. The rats weighting 250 to 300g were caged alone and feed with chow and water.

The irradiation of the rats were performed by using the methods analogous to that as described by John E. Moulder and James J. Fischer (RADIATION REACTION OF RAT SKIN: The role of the Number of Fractions and the Overall Treatment Time. Cancer June 1976;37(6):2762-2767). The rats were anaesthetized with 40 to 50 mg/Kg pentobarbital sodium salt (TCI.TOKYO) intraperitoneally before irradiation. Skin over the gluteus area was shaved completely and the area of the desired radiation of 2 × 2 cm² were marked on the right and left gluteus before irradiation. An electron beam of 6 MeV energy produced by a linear accelerator (Varian Associates, Inc., CA, U.S.A.) was applied for irradiation.

Fractional and single dosage irradiations were tested. In fractional irradiation, three rats were subject to irradiation on the right and left gluteus areas. The radiation was applied twice every week for 5 weeks, and the total dose was 37.5 Gy on each area.

### Dosage and Frequency:

The irradiation injuries in the fractional irradiation were treated with the ointment comprising Fraction 9 (G1) as given in Example 5 after the irradiation once a day at the dosage of 0.25 g/cm², and the irradiation injuries in the single dosage irradiation were treated twice a day. The rats without treatment were used as a control (G2). One blank group (G3) of rats without receiving radiation was as a blank.

### Clinical Evaluation:

The skin reaction scores (Yoshinao Abe and Muneyasu Urano, Fraction side-dependent Acute Skin Reaction of Mice after Multiple Twice-A-Day Doses. Int. J. Radiation Oncology Biol. Phys. 1990 Feb;18(2):359-364; Yu-Jen Chen et al. The Effect of Tetrandrine and Extracts of Centella asiatica on Acute Radiation Dermatitis in Rats. Biol. Pharm. Bull. 1999 July;22(7):703-706) had been evaluated and recorded for 30 days after irradiation. The skin reaction scores were defined as follows: "0" indicating normal skin; "0.5" indicating slight epilation occurring at less than 50% of the radiated area, or discoloration of hair; "1.0" indicating epilation occurring at about 50% of the radiated area; "1.5" indicating epilation occurring at more than 50% of the radiated area and some epilation with red skin; "2.0" indicating complete epilation and dry desquamation occurring at less than 50% of the radiated area; "2.5" indicating dry desquamation occurring at more than 50% of the radiated area; "3.0" indicating some moist desquamation occurring; "3.5" indicating moist desquamation at most of the area.

The skin reaction scores were evaluated and given in Table 8.

**Table 8:**

| Day | skin reaction scores | | |
|---|---|---|---|
| | G1 | G2 | G3 |
| 0 | 0.00 | 0.00 | 0.00 |
| 3 | 0.00 | 0.50 | 0.00 |
| 6 | 0.00 | 0.50 | 0.00 |
| 9 | 0.00 | 0.83 | 0.00 |
| 12 | 0.00 | 0.83 | 0.00 |
| 15 | 0.00 | 0.83 | 0.00 |
| 18 | 0.00 | 0.83 | 0.00 |
| 21 | 0.10 | 0.83 | 0.00 |
| 24 | 0.00 | 0.83 | 0.00 |
| 29 | 0.00 | 1.00 | 0.00 |

In G1, tear early and little hair damage were found in the wounds, and the skin showed no allergy.

The results were similar to those of the fractional irradiation test.

### Histological Evaluation of Skin:

The evaluation methods analogous to those in Example 5 were used.

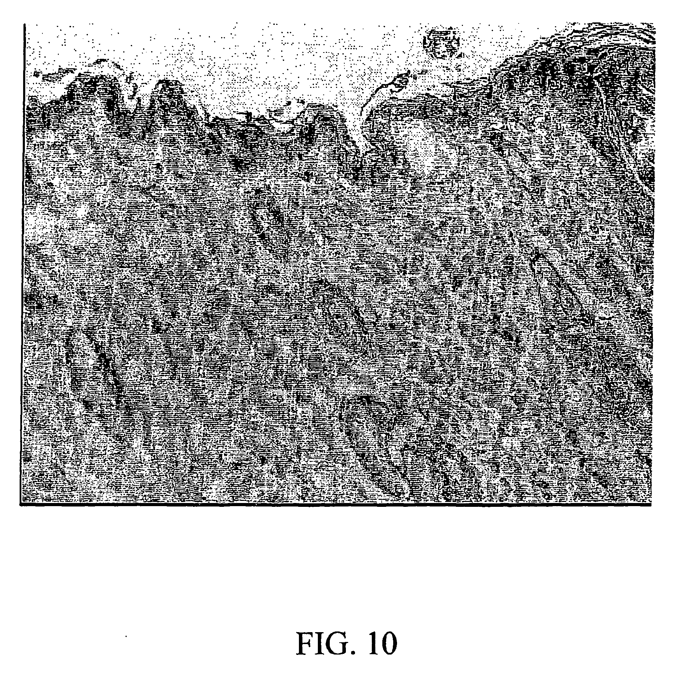

In G1 (as shown in FIG. 10), the epidermal cells were found to cover multi-layered epithelium cells. A large amount of hair follicles were also regenerated.

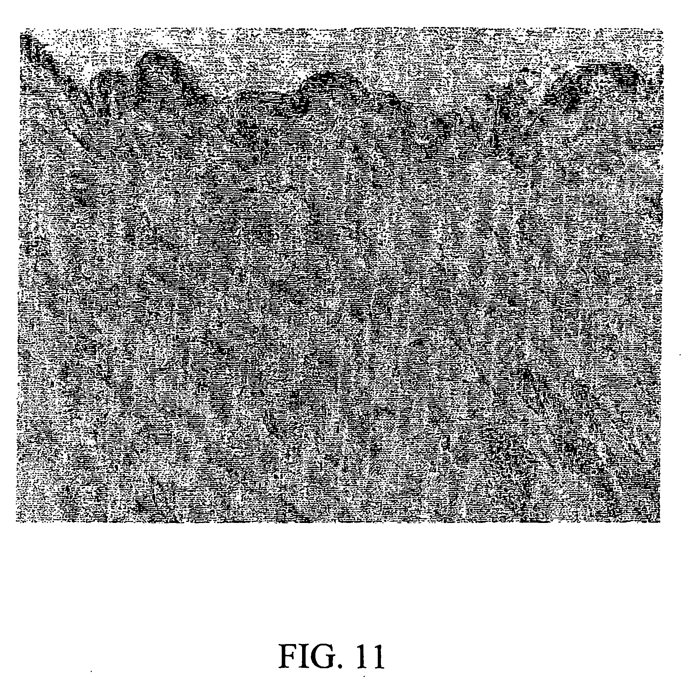

In G2 (as shown in FIG. 11), only small amount of hair follicles were observed.

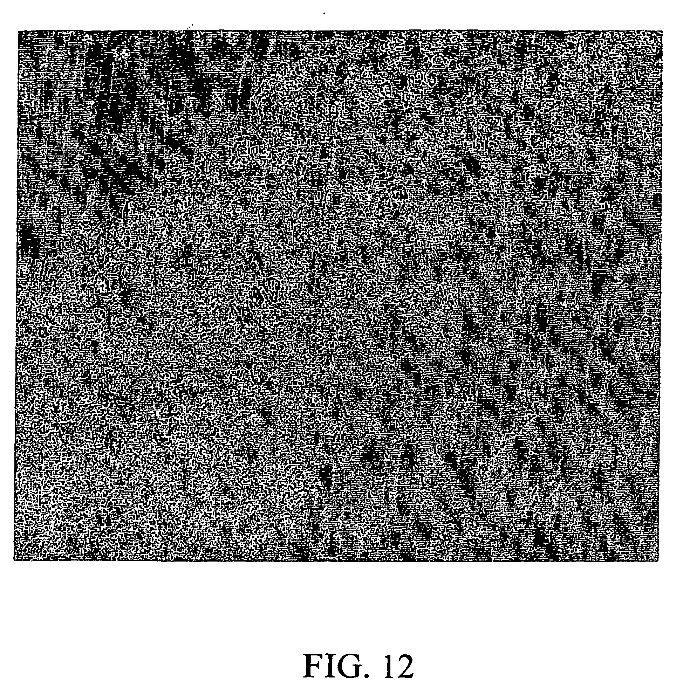

In G3 (as shown in FIG. 12), the tissues were taken as control.

The tissues of the wounds with treatment were dense but those without treatment were loosing.

### Example 8: Effects on Dermatitis on Rabbit Ears

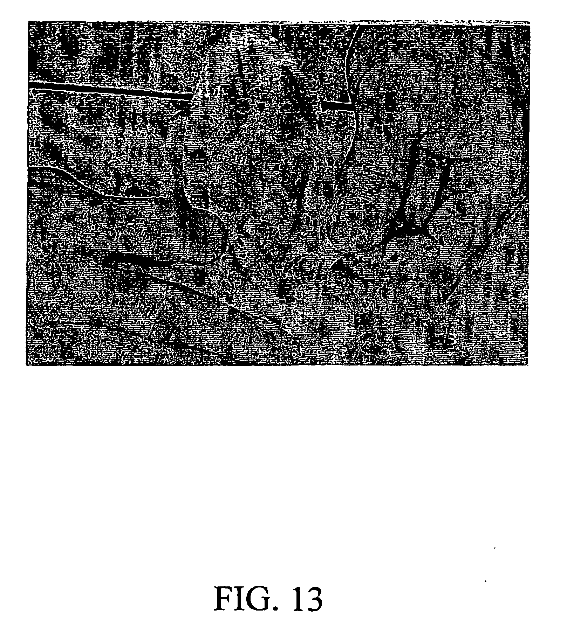

Dermatitis on rabbit ear was found to have symptoms of redness, swell, inflammation, ulcer and bleeding as shown in FIG. 13.

The wounds were applied with the ointment according to the invention that comprises Fraction 10 similar to Fraction 3 except the content of alcohol being 0 % as given in Example 2 for 3 days.

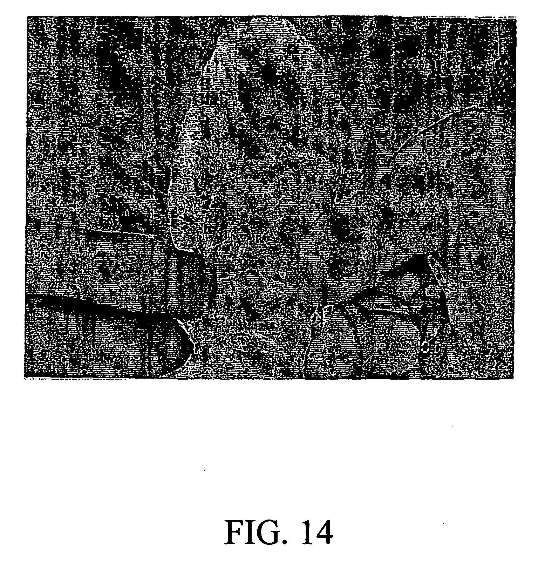

On Day 1, the ulcer disappeared and the redness was alleviated. The hair follicles appeared as shown in FIG. 14.

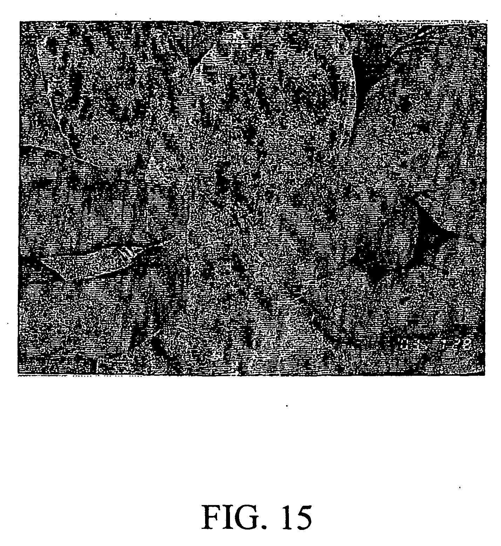

On Day 2, the skin tissue was recovered and only slightly redness was observed. The hair follicles were obviously as shown in FIG. 15.

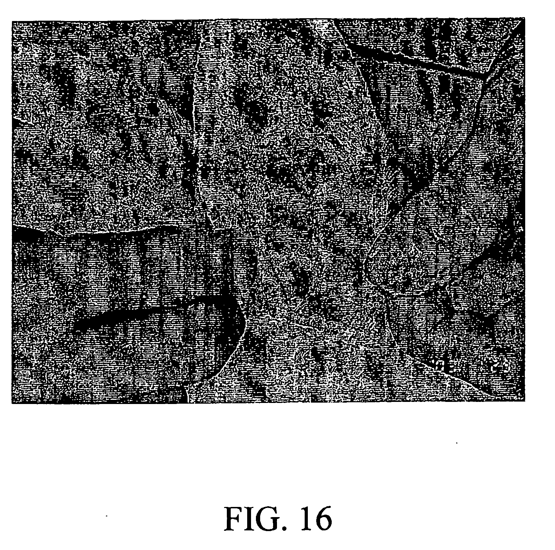

On Day 3, the skin tissue and color were normal. The hair grew on the hair follicles as shown in FIG. 16.

### Example 9: Effects on Diabetes Mellitus Wounds

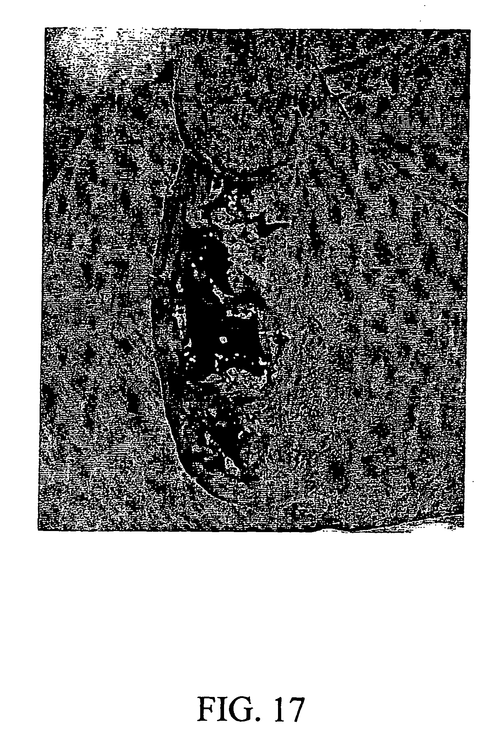

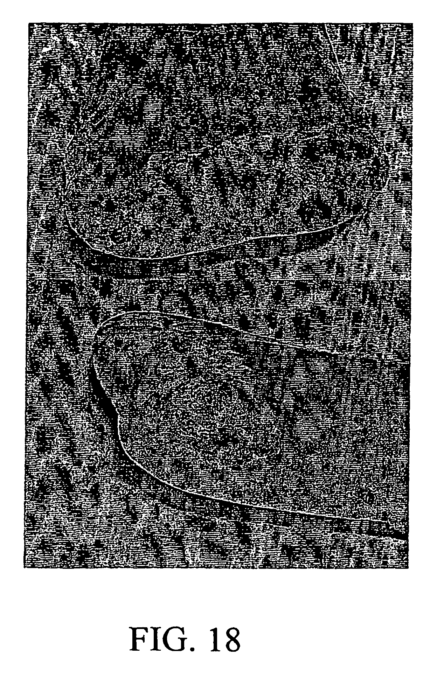

A 68-year-old male had been suffered with diabetes mellitus for 20 years. His toes of left foot were performed with amputation. The wounds showed severe gangrene change as shown in FIG. 17. All toes on his right foot were amputated but the wound healing was not good as shown in FIG. 18.

The ointment comprising Fraction 9 was applied on the wounds 2 to 3 times a day.

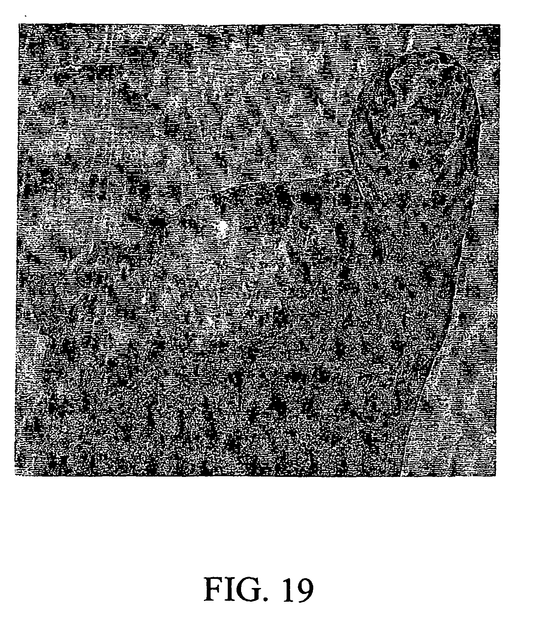

After the treatment of the left foot for 2 months (as shown in FIG. 19), some granulation tissues were noted, and the wound was started to healing without more necrosis.

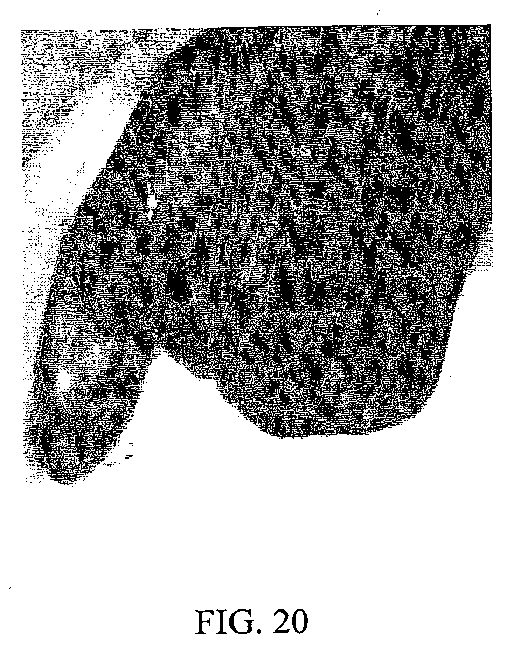

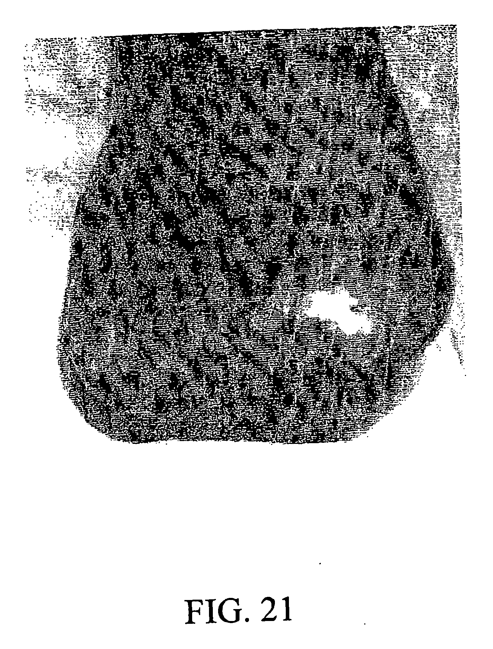

After the treatment of the left foot for 5 months (as shown in FIG. 20), the wound was completely healed without scar. The right foot (as shown in FIG. 21) was also completely healed without scar.

### Example 10: Effects on Chronic Stasis Ulcer

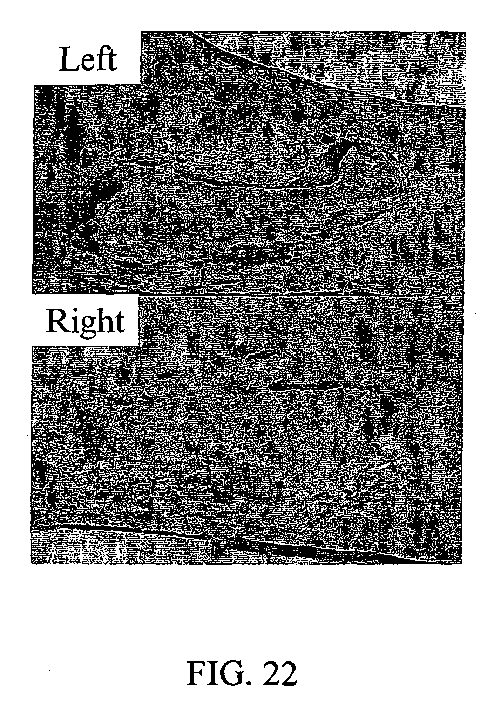

Bilateral lower legs of an 80-year-old male had been suffered with hyperpigmentation and stasis ulcer for 20 years as shown in FIG. 22.

The ointment comprising Fraction 9 was applied on the wounds 2 to 3 times a day.

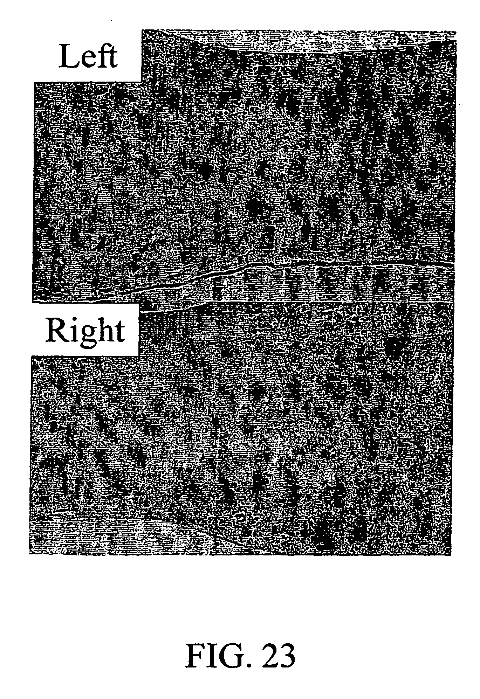

After the treatment of the feet for 2 weeks (as shown in FIG. 23), the wound was started to healing.

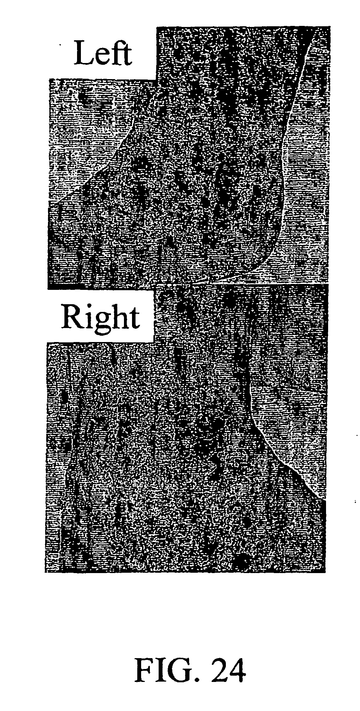

After the treatment of the feet for 4 weeks (as shown in FIG. 24), the wound was completely healed without scar.

### Example 11: Effects on Ecchymosis

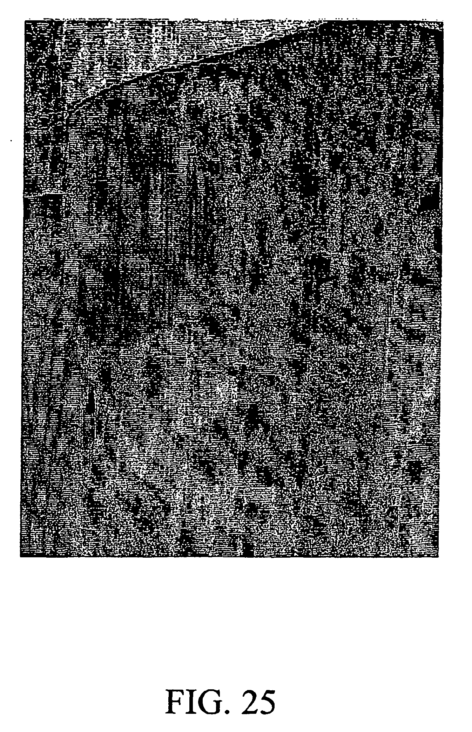

A female patient had ecchymosis caused by an accident (as shown in FIG. 25).

The wounds were pretreated with a cold compress once a day for three days, and then applied with the ointment comprising Fraction 9 2 to 3 times a day.

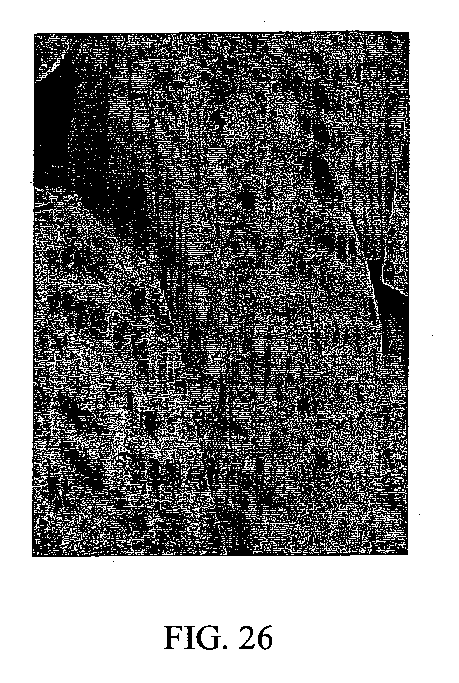

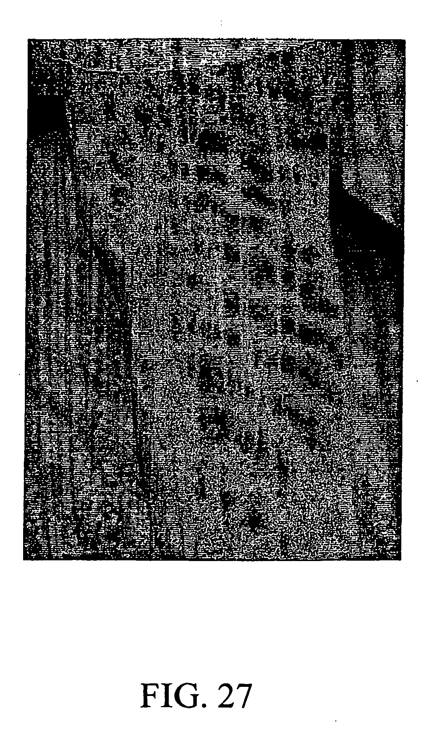

The ecchymosis disappeared and the wounds were healed on the third day post treatment as shown in FIG. 26. The color of the scar that was already present before the car accident was also flattened and lightened after treating for 7 days (as shown in FIG. 27).

### Example 12: Effects on Abrasion

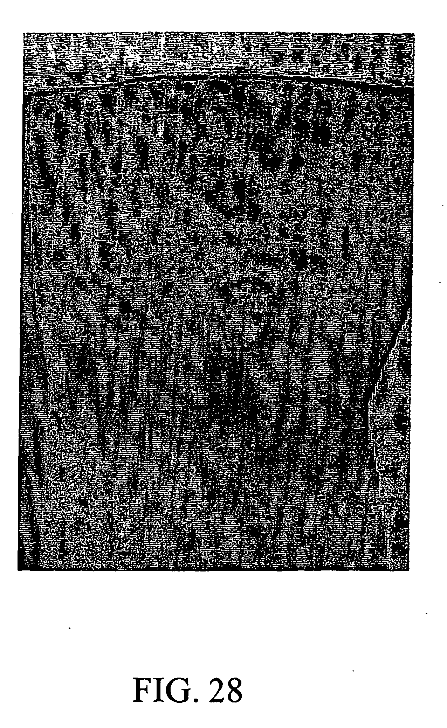

A female patient had skin abrasion caused by an accident as shown in FIG. 28.

The ointment comprising Fraction 9 was applied on the wounds 2 to 3 times a day.

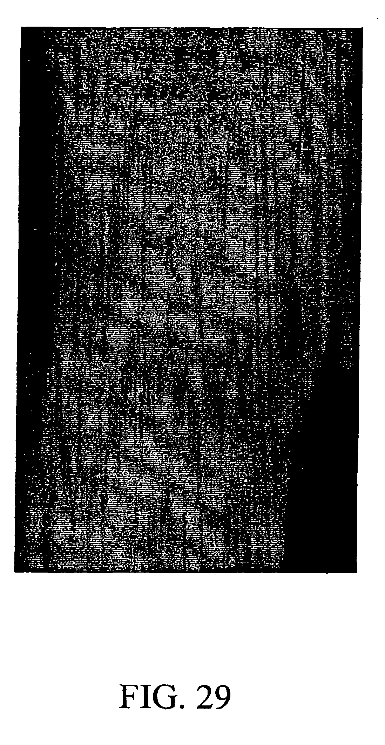

The wounds were healed after the treatment for 7 days (as shown in FIG. 29).

### Example 13: Effects on Suture Wounds

A male patient had serious laceration wounds on the philtrum with 3 cm break and 20-stitches suture, nose beam with 0.8 cm and 4-stitches suture and upper lip with 1 cm break and 6-stitches suture and 1.5 cm break and 8-stitches suture.

The ointment comprising Fraction 9 was applied on the wounds 2 to 3 times a day.

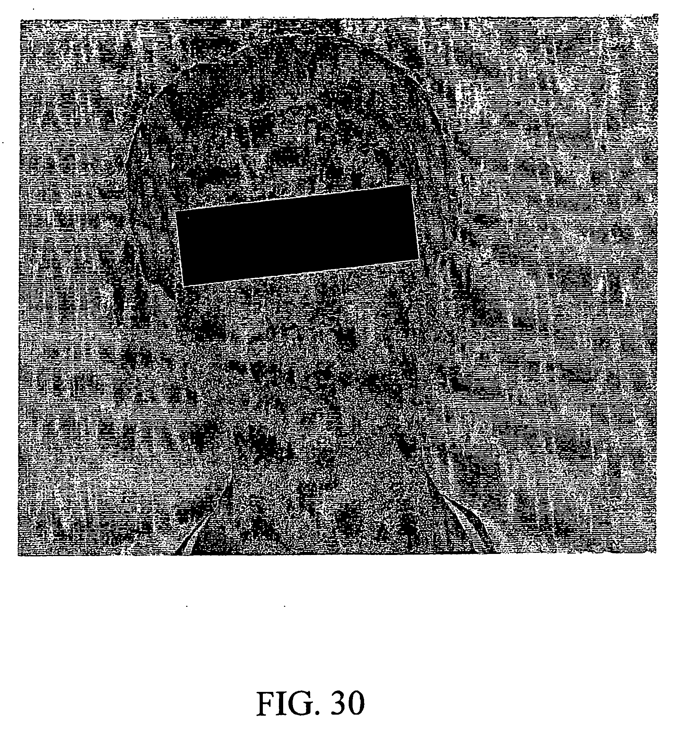

The wounds were healed completely without scar (as shown in FIG. 30).

### Example 14: Effects on Atopic Dermatitis

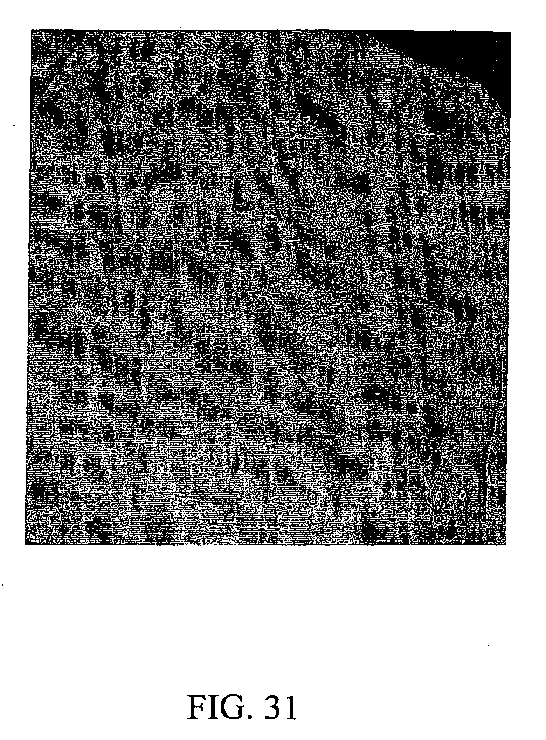

A 4.5-year-old female had chronic atopic dermatitis. The skin of the neck and upper thorax was itchy, redness, swelling, crackling, weeping, crusting and scaling after long-term topical corticosteroid therapy as shown in FIG. 31.

The ointment comprising Fraction 10 was applied on the wounds 2 to 3 times a day.

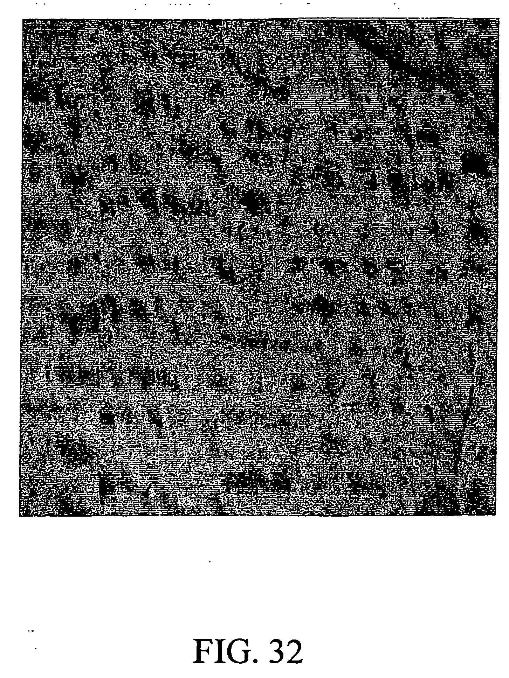

After the treatment for 1 week (as shown in FIG. 32), the itching, redness, swelling, cracking, weeping, crusting, and scaling were reduced and dectreased.

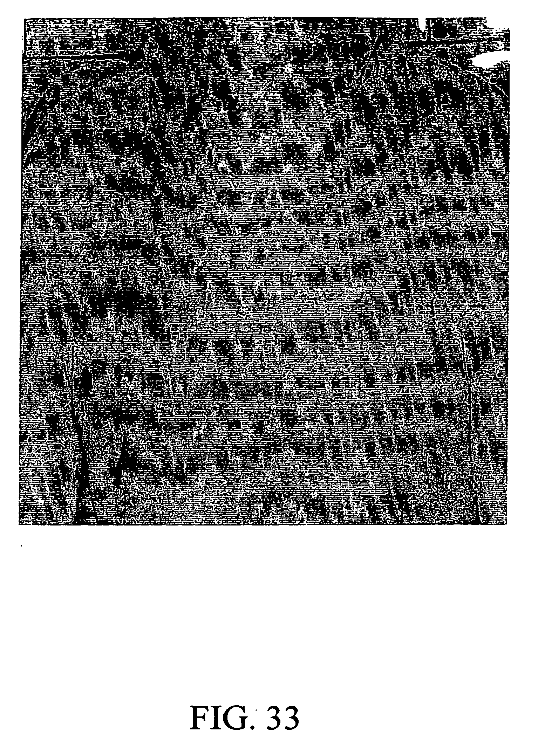

After the treatment for 4 weeks (as shown in FIG. 33), the skin lesions were almost subsided.

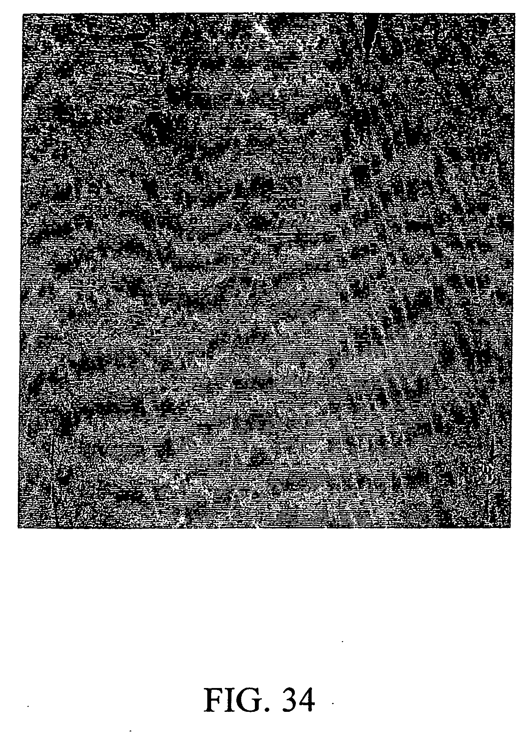

After the treatment for 6 weeks (as shown in FIG. 34), the skin lesions were completely healed with no rebound effect later.

While embodiments of the present invention have been illustrated and described, various modifications and improvements can be made by persons skilled in the art. The embodiments of the present invention are therefore described in an illustrative but not restrictive sense.

## Claims

1. A vapor fraction from seeds of *Glycine max* (L.) Merr., which vapor fraction is obtainable by a process comprising:
(a) providing a crude extract of the seeds in an alcohol solution containing alcohol at a concentration lower than about 15% wt. or in water; and
(b) vaporizing the crude extract at a barometric pressure lower than about 1 atm and at a temperature lower than about 110°C to obtain the vapor fraction;
wherein a spectrogram of 50 µL of vapor fraction taken at 200 nm through a high performance liquid chromatography (HPLC) using Waters Sphersorb^{™}-ODS2 column with an inner diameter (LD.) of 4.6 mm, a length (L) of 250 mm and a particle size of 5 µm comprises peaks at a retention time of 2.910, 5.190, 13.190, and 50.815 minutes with the coefficient of variation of 8%, where mobile phase at 0 to 5 minutes is 95% H₂0/5% CH₃CN; at 5 to 30 minutes is 95% H₂O/5% CH₃CN in gradient; at 30 to 80 minutes is 75% H₂O/25% CH₃CN in gradient; at 80 to 100 minutes is 0% H₂O/100% CH₃CN in gradient; at 100 to 101 minutes is 0% H₂O/100% CH₃CN in gradient; and at 101 minute is 95% H₂O/5% CH₃CN and the flow rate is 1 mL/min.

2. A vapor fraction from the raw materials comprising seeds of *Glycine max* (L.) Merr. and at least one of seeds of *Sesamum indicum,* seeds of *Phaseolus radiatus* L. or material from an antimicrobial herb, which vapor fraction is obtainable by a process comprising:
(a) providing a crude extract of the raw materials in an alcohol solution containing alcohol at a concentration lower than about 15% wt. or in water; and
(b) vaporizing the crude extract at a barometric pressure lower than about 1 atm and at a temperature lower than about 110°C to obtain the vapor fraction;
wherein a spectrogram of 50 µL of vapor fraction taken at 200 nm through a high performance liquid chromatography (HPLC) using Waters Sphersorb^{™}-ODS2 column with an inner diameter (LD.) of 4.6 mm, a length (L) of 250 mm and a particle size of 5 µm comprises peaks at a retention time of 2.910, 5.190, 13.190, and 50.815 minutes with the coefficient of variation of 8%, where mobile phase at 0 to 5 minutes is 95% H₂0/5% CH₃CN; at 5 to 30 minutes is 95% H₂O/5% CH₃CN in gradient; at 30 to 80 minutes is 75% H₂O/25% CH₃CN in gradient; at 80 to 100 minutes is 0% H₂O/100% CH₃CN in gradient; at 100 to 101 minutes is 0% H₂O/100% CH₃CN in gradient; and at 101 minute is 95% H₂O/5% CH₃CN and the flow rate is 1mL/min.

3. The vapor fraction according to claim 2, wherein the material from an antimicrobial herb is dry rhizomes of *Rheum palmatum* L., dry rhizomes of Rhei Rhizoma, dry barks of *Phellodendron amurense Rupr.,* or dry barks of Phellodendri Cortex.

4. The vapor fraction according to claim 2, wherein the raw materials comprise about 40 to 100% of seeds of *Glycine max* (L.) Merr., 0 to about 50% seeds of *Sesamum indicum,* 0 to about 50% of seeds of *Phaseolus radiatus* L. and 0 to about 30% of material from an antimicrobial herb.

5. The vapor fraction according to any one of the preceding claims, wherein the vapor fraction is collected in a liquid form by chilling the vapor.

6. The vapor fraction according to any one of the preceding claims, wherein the concentration of alcohol is lower than about 5% wt.

7. The vapor fraction according to any one of the preceding claims, wherein the crude fraction in the step (a) is in water.

8. The vapor fraction according to any one of the preceding claims, wherein the temperature in the step (b) ranges from about 60°C to about 110°C.

9. The vapor fraction according to any one of the preceding claims, for use in treating skin injuries or dermatological disorders or for stimulating cell regeneration or stimulating hair growth.

10. Use of the vapor fraction according to any of claims 1 to 8 in the manufacture of a medicament for use in treating skin injuries or dermatological disorders or for stimulating cell regeneration or stimulating hair growth.

11. The use according to claim 10 wherein the skin injuries or dermatological disorders are burn injury, sunburns, irradiation injury, acute and chronic trauma, ecchymosis, dermatitis, macule, papule, nodule, vesicle, bulla, pustule, wheal, plaque, cyst, scales, crust, fissure, ulcer, acne, xeroderma, desquamation, itch, allergic dermatitis, exanthema, gangrene, chronic stasis ulcer, abrasion, atopic dermatitis, suture wounds, diabetes mellitus wounds, or hair follicle defect.

12. Use according to claim 10 or 11, wherein the medicament comprises borneol, stearic acid, stearyl alcohol, palmitic acid, cetyl alcohol, beeswax and/or polyoxy ethylene sorbitan monostearate.

13. A composition comprising the vapor fraction according to any one of claims 1 to 8.

14. The composition according to claim 13, which is a pharmaceutical composition, a cosmetic composition or a skin cleaning composition.

15. The composition according to claims 13 or 14, which comprises a pharmaceutically acceptable carrier or diluent.

16. The composition according to any one of claims 13 to 15 which comprises an antimicrobial agent.

17. The composition according to anyone of claims 13 to 16 which comprises borneol, stearic acid, stearyl alcohol, palmitic acid, cetyl alcohol, beeswax and/or polyoxy ethylene sorbitan monostearate.

18. A process for the preparation of a vapor fraction from seeds of *Glycine max* (L.) Merr., which process comprises:
(a) providing a crude extract of the seeds in an alcohol solution containing alcohol at a concentration lower than about 15% wt. or in water; and
(b) vaporizing the crude extract at a barometric pressure lower than about 1 atm and at a temperature lower than about 110°C to obtain the vapor fraction.

19. A process for the preparation of a vapor fraction from the raw materials comprising seeds of *Glycine max* (L.) Merr. and at least one of seeds of *Sesamum indicum,* seeds of *Phaseolus radiatus* L. or a material from an antimicrobial herb, which process comprises:
(a) providing a crude extract of the raw materials in an alcohol solution containing alcohol at a concentration lower than about 15% wt. or in water; and
(b) vaporizing the crude extract at a barometric pressure lower than about 1 atm and at a temperature lower than about 110°C to obtain the vapor fraction.

20. The vapor fraction according to claim 19, wherein the material from an antimicrobial herb is dry rhizomes of *Rheum palmatum* L., dry rhizomes of Rhei Rhizoma, dry barks of *Phellodendron amurense Rupr.,* or dry barks of Phellodendri Cortex.

21. The vapor fraction according to claim 19, wherein the raw materials comprise about 40 to about 100% of seeds of *Glycine max* (L.) Merr., 0 to about 50% of seeds of *Sesamum indicum,* 0 to about 50% of seeds of *Phaseolus radiatus* L. and 0 to about 30% of a material from an antimicrobial herb.

22. The process according to any one of claims 18 to 21, wherein the vapor fraction is collected in a liquid form by chilling the vapor.

23. The process according to any one of claims 18 to 22, wherein the concentration of alcohol is lower than about 5% wt.

24. The process according to any one of claims 18 to 23, wherein the crude fraction in the step (a) is in water.

25. The process according to any one of claims 18 to 24, wherein the temperature in the step (b) ranges from about 60°C to about 110°C.

## Patentansprüche

1. Dampffraktion aus Samen von Glycine max (L.) Merr., die erhältlich ist durch ein Verfahren, das folgendes umfasst:
(a) Bereitstellen eines rohen Extrakts der Samen in einer Alkohollösung mit einem Gehalt an Alkohol in einer Konzentration von weniger als etwa 15 Gew.-% oder in Wasser; und
(b) Verdampfen des rohen Extrakts bei einem barometrischen Druck unter etwa 1 atm und einer Temperatur unter etwa 110 °C, um die Dampffraktion zu erhalten;
wobei ein Spektrogramm von 50 µl der Dampffraktion, das bei 200 nm durch Hochleistungs-Flüssigchromatographie (HPLC) unter Verwendung einer Waters Sphersorb^{™}-ODS2-Säule mit einem Innendurchmesser (ID) von 4,6 mm, einer Länge (L) von 250 mm und einer Teilchengröße von 5 µm aufgenommen worden ist, Peaks bei einer Retentionszeit von 2,910, 5,190, 13,190 und 50,815 Minuten bei einem Variationskoeffizienten von 8 % umfasst, wobei es sich bei der mobilen Phase von 0 bis 5 Minuten um 95 % H₂O/5 % CH₃CN; von 5 bis 30 Minuten um 95 % H₂O/5 % CH₃CN als Gradient; von 30 bis 80 Minuten um 75 % H₂O/25 % CH₃CN als Gradient; von 80 bis 100 Minuten um 0 % H₂O/100 % CH₃CN als Gradient; von 100 bis 101 Minuten um 0 % H₂O/100 % CH₃CN als Gradient; und bei 101 Minuten um 95 % H₂O/5 % CH₃CN handelt und die Fließgeschwindigkeit 1 ml/min beträgt.

2. Dampffraktion aus Rohmaterialien, die Samen von Glycine max (L.) Merr. und mindestens einen der Samen von Sesamum indicum, Samen von Phaseolus radiatus L. oder Material aus einer antimikrobiellen Kräuterpflanze umfasst, wobei die Dampffraktion durch ein Verfahren erhältlich ist, das folgendes umfasst:
(a) Bereitstellen eines rohen Extrakts der Rohmaterialien in einer Alkohollösung mit einem Gehalt an Alkohol in einer Konzentration von weniger als etwa 15 Gew.-% oder in Wasser; und
(b) Verdampfen des rohen Extrakts bei einem barometrischen Druck unter etwa 1 atm und einer Temperatur unter etwa 110 °C, um die Dampffraktion zu erhalten;
wobei ein Spektrogramm von 50 µl der Dampffraktion, das bei 200 nm durch Hochleistungs-Flüssigchromatographie (HPLC) unter Verwendung einer Waters Sphersorb^{™}-ODS2-Säule mit einem Innendurchmesser (ID) von 4,6 mm, einer Länge (L) von 250 mm und einer Teilchengröße von 5 µm aufgenommen worden ist, Peaks bei einer Retentionszeit von 2,910, 5,190, 13,190 und 50,815 Minuten bei einem Variationskoeffizienten von θ % umfasst, wobei es sich bei der mobilen Phase von 0 bis 5 Minuten um 95 % H₂O/5 % CH₃CN; von 5 bis 30 Minuten um 95 % H₂O/5 % CH₃CN als Gradient; von 30 bis 80 Minuten um 75 % H₂O/25 % CH₃CN als Gradient; von 80 bis 100 Minuten um 0 % H₂O/100 % CH₃CN als Gradient; von 100 bis 101 Minuten um 0 % H₂O/100 % CH₃CN als Gradient; und bei 101 Minuten um 95 % H₂O/5 % CH₃CN handelt und die Fließgeschwindigkeit 1 ml/min beträgt.

3. Dampffraktion nach Anspruch 2, wobei es sich bei dem Material aus einer antimikrobiellen Kräuterpflanze um trockene Rhizome von Rheum palmatum L., trockene Rhizome von Rhei Rhizoma, trockene Rinden von Phellodendron amurense Rupr. oder trockene Rinden von Phellodendri Cortex handelt.

4. Dampffraktion nach Anspruch 2, wobei die Rohmaterialien etwa 40 bis 100 % Samen von Glycine max (L.) Merr., 0 bis etwa 50 % Samen von Sesamum indicum, 0 bis etwa 50 % Samen von Phaseolus radiatus L. und 0 bis etwa 30 % Material aus einer antimikrobiellen Kräuterpflanze umfasst.

5. Dampffraktion nach einem der vorstehenden Ansprüche, wobei die Dampffraktion in einer flüssigen Form durch Abkühlen des Dampfes gewonnen wird.

6. Dampffraktion nach einem der vorstehenden Ansprüche, wobei die Alkoholkonzentration niedriger als etwa 5 Gew,-% ist,

7. Dampffraktion nach einem der vorstehenden Ansprüche, wobei die rohe Fraktion in Stufe (a) in Wasser vorliegt.

8. Dampffraktion nach einem der vorstehenden Ansprüche, wobei die Temperatur in Stufe (b) im Bereich von etwa 60 bis etwa 110 °C liegt.

9. Dampffraktion nach einem der vorstehenden Ansprüche zur Verwendung bei der Behandlung von Hautschädigungen oder dermatologischen Störungen oder zur Stimulation der Zellregeneration oder zur Stimulation des Haarwachstums.

10. Verwendung der Dampffraktion nach einem der Ansprüche 1 bis 8 bei der Herstellung eines Arzneimittels zur Verwendung bei der Behandlung von Hautschädigungen oder dermatologischen Störungen oder zur Stimulation der Zellregeneration oder zur Stimulation des Haarwachstums.

11. Verwendung nach Anspruch 10, wobei es sich bei den Hautschädigungen oder dermatologischen Störungen um Brandverletzungen, Sonnenbrand, Strahlungsverletzungen, akute und chronische Traumata, Ekchymose, Dermatitis, Flecken, Papeln, Knötchen, Vesikel, Bläschen, Pusteln, Quaddeln, Plaques, Zysten, Schuppen, Krusten, Schrunden, Geschwüre, Akne, Xerodermie, Abschuppungen, Jucken, allergische Dermatitis, Exanthem, Gangrän, chronisches Stauungsgeschwür, Abrieb, atopische Dermatitis, Nahtwunden, Diabetes mellitus-Wunden oder Haarfollikeldefekte handelt.

12. Verwendung nach Anspruch 10 oder 11, wobei das Arzneimittel Borneol, Stearinsäure, Stearylalkohol, Palmitinsäure, Cetylalkohol, Bienenwachs und/oder Polyoxyethylensorbitanmonostearat umfasst.

13. Zusammensetzung, umfassend die Dampffraktion nach einem der Ansprüche 1 bis 8.

14. Zusammensetzung nach Anspruch 13, wobei es sich um eine pharmazeutische Zusammensetzung, eine kosmetische Zusammensetzung oder eine Hautreinigungszusammensetzung handelt.

15. Verfahren nach Anspruch 13 oder 14, umfassend einen pharmazeutisch verträglichen Träger oder Verdünnungsmittel.

16. Zusammensetzung nach einem der Ansprüche 13 bis 15, umfassend ein antimikrobielles Mittel.

17. Zusammensetzung nach einem der Ansprüche 13 bis 16, umfassend Borneol, Stearinsäure, Stearylalkohol, Palmitinsäure, Cetylalkohol, Bienenwachs und/oder Polyoxyethylensorbitanmonostearat.

18. Verfahren zur Herstellung einer Dampffraktion aus Samen von Glycine max (L.) Merr., wobei das Verfahren folgendes umfasst:
(a) Bereitstellen eines rohen Extrakts der Samen in einer Alkohollösung mit einem Gehalt an Alkohol in einer Konzentration von: weniger als etwa 15 Gew.-% oder in Wasser; und
(b) Verdampfen des rohen Extrakts bei einem barometrischen Druck unter etwa 1 atm und einer Temperatur unter etwa 110 °C, um die Dampffraktion zu erhalten.

19. Verfahren zur Herstellung einer Dampffraktion aus Rohmaterialien, die Samen von Glycine max (L.) Merr. und mindestens einen der Samen von Sesamum indicum, Samen von Phaseolus radiatus L. oder ein Material aus einer antimikrobiellen Kräuterpflanze umfasst, wobei das Verfahren folgendes umfasst:
(a) Bereitstellen eines rohen Extrakts der Rohmaterialien in einer Alkohollösung mit einem Gehalt an Alkohol in einer Konzentration von weniger als etwa 15 Gew.-% oder in Wasser; und
(b) Verdampfen des rohen Extrakts bei einem barometrischen Druck unter etwa 1 atm und einer Temperatur unter etwa 110 °C, um die Dampffraktion zu erhalten.

20. Dampffraktion nach Anspruch 19, wobei es sich bei dem Material aus einer antimikrobiellen Kräuterpflanze um trockene Rhizome von Rheum palmatum L., trockene Rhizome von Rhei Rhizoma, trockene Rinden von Phellodendron amurense Rupr. oder trockene Rinden von Phellodendri Cortex handelt.

21. Dampffraktion nach Anspruch 19, wobei die Rohmaterialien etwa 40 bis 100 % Samen von Glycine max (L.) Merr., 0 bis etwa 50 % Samen von Sesamum indicum, 0 bis etwa 50 % Samen von Phaseolus radiatus L. und 0 bis etwa 30 % Material aus einer antimikrobiellen Kräuterpflanze umfasst.

22. Verfahren nach einem der Ansprüche 18 bis 21, wobei die Dampffraktion in einer flüssigen Form durch Abkühlen des Dampfes gewonnen wird.

23. Verfahren nach einem der Ansprüche 18 bis 22, wobei die Alkoholkonzentration niedriger als etwa 5 Gew.-% ist.

24. Verfahren nach einem der Ansprüche 18 bis 23, wobei die rohe Fraktion in Stufe (a) in Wasser vorliegt.

25. Verfahren nach einem der Ansprüche 18 bis 24, wobei die Temperatur in Stufe (b) im Bereich von etwa 60 bis etwa 110 °C liegt.

## Revendications

1. Une fraction vapeur de graines de Glycine max (L.) Merr., laquelle fraction vapeur est obtenable par un procédé comprenant :
(a) la fourniture d'un extrait brut des graines dans une solution d'alcool contenant de l'alcool à une concentration inférieure à environ 15 % en poids ou dans l'eau ; et
(b) la vaporisation de l'extrait brut à une pression barométrique inférieure à environ 1 atm et à une température inférieure à environ 110° C afin d'obtenir la fraction vapeur ;
dans lequel un spectrogramme de 50 µL de fraction vapeur pris à 200 nm par une chromatographie liquide à haute performance (HPLC) utilisant une colonne Waters Sphersorb^{™}-ODS2 avec un diamètre intérieur (I.D.) de 4,6 mm, une longueur (L) de 250 mm et une taille de particules de 5 µm comprend des pics à un temps de rétention de 2,910, 5,190, 13,190, et 50,815 minutes avec le coefficient de variation de 8 %, où la phase mobile de 0 à 5 minutes est 95 % H₂0/5 % CH₃CN ; de 5 à 30 minutes est 95 % H₂O/5 % CH₃CN en gradient ; de 30 à 80 minutes est 75 % H₂O/2 % CH₃CN en gradient ; de 80 à 100 minutes est 0 % H₂O/100 % CH₃CN en gradient ; de 100 à 101 minutes est 0 % H₂O/100 % CH₃CN en gradient ; et de 101 minute est 95 % H₂O/5 % CH₃CN et le débit est 1 mL/min.

2. Une fraction vapeur à partir des matières brutes comprenant des graines de Glycine max (L.) Merr. et au moins une des graines de Sesamum indicum, des graines de Phaseolus radiatus L. ou de la matière d'une herbe antimicrobienne, laquelle fraction vapeur est obtenable par un procédé comprenant :
(a) la fourniture d'un extrait brut des matières brutes dans une solution d'alcool contenant de l'alcool à une concentration inférieure à environ 15 % en poids ou dans l'eau ; et
(b) la vaporisation de l'extrait brut à une pression barométrique inférieure à environ 1 atm et à une température inférieure à environ 110° C afin d'obtenir la fraction vapeur ;
dans lequel un spectrogramme de 50 µL de fraction vapeur pris à 200 nm par une chromatographie liquide à haute performance (HPLC) utilisant une colonne Waters Sphersorb^{™}-ODS2 avec un diamètre intérieur (I.D.) de 4,6 mm, une longueur (L) de 250 mm et une taille de particules de 5 µm comprend des pics à un temps de rétention de 2,910, 5,190, 13,190, et 50,815 minutes avec le coefficient de variation de 8 %, où la phase mobile de 0 à 5 minutes est 95 % H₂O/5 % CH₃CN ; de 5 à 30 minutes est 95 % H₂O/5 % CH₃CN en gradient ; de 30 à 80 minutes est 75 % H₂O/25 % CH₃CN en gradient ; de 80 à 100 minutes est 0 % H₂O/100 % CH₃CN en gradient ; de 100 à 101 minutes est 0 % H₂O/100 % CH₃CN en gradient ; et de 101 minute est 95 % H₂O/5 % CH₃CN et le débit est 1 mL/min.

3. La fraction vapeur selon la revendication 2, dans laquelle les matières d'une herbe antimicrobienne sont des rhizomes secs de Rheum palmatum L., des rhizomes secs de Rhei Rhizoma, des écorces sèches de Phellodendron amurense Rupr., ou des écorces sèches de Phellodendri Cortex.

4. La fraction vapeur selon la revendication 2, dans laquelle les matières brutes comprennent environ 40 à 100 % de graines de Glycine max (L.) Merr., 0 à environ 50 % de graines de Sesamum indicum, 0 à environ 50 % de graines de Phaseolus radiatus L. et 0 à environ 30 % de matière d'une herbe antimicrobienne.

5. La fraction vapeur selon l'une quelconque des revendications précédentes, dans laquelle la fraction vapeur est collectée sous forme de liquide en réfrigérant la vapeur.

6. La fraction vapeur selon l'une quelconque des revendications précédentes, dans laquelle la concentration de l'alcool est inférieure à environ 5 % en poids.

7. La fraction vapeur selon l'une quelconque des revendications précédentes, dans laquelle la fraction brute à l'étape (a) est dans l'eau.

8. La fraction vapeur selon l'une quelconque des revendications précédentes, dans laquelle la température à l'étape (b) se situe dans une gamme allant d'environ 60° C à environ 110° C.

9. La fraction vapeur selon l'une quelconque de revendications précédentes, pour utilisation dans le traitement de blessures de la peau ou de troubles dermatologiques ou pour la stimulation de la régénération de cellules ou la stimulation de la croissance de cheveux.

10. Utilisation de la fraction vapeur selon l'une quelconque des revendications 1 à 8 pour la fabrication d'un médicament pour utilisation dans le traitement de blessures de la peau ou de troubles dermatologiques ou pour la stimulation de la régénération de cellules ou la stimulation de la croissance de cheveux.

11. L'utilisation selon la revendication 10 dans laquelle les blessures de la peau ou les troubles dermatologiques sont une blessure de brûlure, des coups de soleil, une blessure par irradiation, des traumatismes aigus et chroniques, une ecchymose, une dermatite, une macule, une papule, un nodule, une vésicule, une bulle, une pustule, une plaque ortiée, un placard, un kyste, des squames, une croûte, une fissure, un ulcère, une acné, une xérodermie, une desquamation, une démangeaison, une dermatite allergique, un exanthème, une gangrène, un ulcère variqueux chronique, une abrasion, une dermatite atopique, des blessures de suture, des blessures de diabète sucré, ou un défaut de follicule pileux.

12. Utilisation selon la revendication 10 ou 11, dans laquelle le médicament comprend du bornéol, de l'acide stéarique, de l'alcool stéarylique, de l'acide palmitique, de l'alcool cétylique, de la cire d'abeille et/ou du monostéarate de sorbitan de polyoxyéthylène.

13. Une composition comprenant la fraction vapeur selon l'une quelconque des revendications 1 à 8.

14. La composition selon la revendication 13, qui est une composition pharmaceutique, une composition cosmétique ou une composition nettoyante pour la peau.

15. La composition selon les revendications 13 ou 14, qui comprend un vecteur ou un diluant acceptable pharmaceutiquement.

16. La composition selon l'une quelconque des revendications 13 à 15 qui comprend un agent antimicrobien.

17. La composition selon l'une quelconque des revendications 13 à 16 qui comprend du bornéol, de l'acide stéarique, de l'alcool stéarylique, de l'acide palmitique, de l'alcool cétylique, de la cire d'abeille et/ou du monostéarate de sorbitan de polyoxyéthylène.

18. Un procédé pour la préparation d'une fraction vapeur de graines de Glycine max (L.) Merr., lequel procédé comprend:
(a) la fourniture d'un extrait brut des graines dans une solution d'alcool contenant de l'alcool à une concentration inférieure à environ 15 % en poids ou dans l'eau ; et
(b) la vaporisation de l'extrait brut à une pression barométrique inférieure à environ 1 atm et à une température inférieure à environ 110° C pour obtenir la fraction vapeur.

19. Un procédé pour la préparation d'une fraction vapeur des matières brutes comprenant des graines de Glycine max (L.) Merr. et au moins une des graines de Sesamum indicum, des graines de Phaseolus radiatus L. ou une matière d'une herbe antimicrobienne, lequel procédé comprend :
(a) la fourniture d'un extrait brut des matières brutes dans une solution d'alcool contenant de l'alcool à une concentration inférieure à environ 15 % en poids ou dans l'eau ; et
(b) la vaporisation de l'extrait brut à une pression barométrique inférieure à environ 1 atm et à une température inférieure à environ 110° C pour obtenir la fraction vapeur.

20. La fraction vapeur selon la revendication 19, dans laquelle les matières d'une herbe antimicrobienne sont des rhizomes secs de Rheum palmatum L., des rhizomes secs de Rhei Rhizoma, des écorces sèches de Phellodendron amurense Rupr., ou des écorces sèches de Phellodendri Cortex.

21. La fraction vapeur selon la revendication 19, dans laquelle les matières brutes comprennent environ 40 à environ 100 % de graines de Glycine max (L.) Merr., 0 à environ 50 % de graines de Sesamum indicum, 0 à environ 50 % de graines de Phaseolus radiatus L. et 0 à environ 30 % d'une matière d'une herbe antimicrobienne.

22. Le procédé selon l'une quelconque des revendications 18 à 21, dans lequel la fraction vapeur est collectée sous forme liquide en réfrigérant la vapeur.

23. Le procédé selon l'une quelconque des revendications 18 à 22, dans lequel la concentration d'alcool est inférieure à environ 5 % en poids.

24. Le procédé selon l'une quelconque des revendications 18 à 23, dans laquelle la fraction brute à l'étape (a) est dans l'eau.

25. Le procédé selon l'une quelconque des revendications 18 à 24, dans laquelle la température à l'étape (b) se situe dans une gamme allant d'environ 60° C à environ 110° C.
